(19) Europäisches Patentamt
European Patent Office
Office européen des brevets

(11) Veröffentlichungsnummer: **0 475 204 A2**

# (12) EUROPÄISCHE PATENTANMELDUNG

(21) Anmeldenummer: **91114518.3**

(51) Int. Cl.5: **C07D 495/14**, A61K 31/38

(22) Anmeldetag: **29.08.91**

(30) Priorität: **30.08.90 DE 4027470**

(43) Veröffentlichungstag der Anmeldung:
**18.03.92 Patentblatt 92/12**

(84) Benannte Vertragsstaaten:
**AT BE CH DE DK ES FR GB GR IT LI LU NL SE**

(71) Anmelder: **BOEHRINGER INGELHEIM KG**

**W-6507 Ingelheim am Rhein(DE)**
(84) **BE CH DE DK ES FR GR IT LI LU NL SE AT**

(71) Anmelder: **BOEHRINGER INGELHEIM INTERNATIONAL G.M.B.H.**

**W-6507 Ingelheim am Rhein(DE)**
(84) **GB**

(72) Erfinder: **Weber, Karl-Heinz, Dr.**
**Kaiser-Karl-Strasse 11**
**W-6535 Gau-Algesheim(DE)**
Erfinder: **Stransky, Werner, Dr.**
**Im Hippel 24**
**W-6535 Gau-Algesheim(DE)**
Erfinder: **Küfner-Mühl, Ulrike, Dr.**
**Am Rübenacker 5**
**W-6500 Mainz(DE)**
Erfinder: **Heuer, Hubert, Dr.**
**Am Sportfeld 74**
**W-6501 Schwabenheim(DE)**
Erfinder: **Birke, Franz, Dr.**
**Albrecht-Dürer-Strasse 23**
**W-6507 Ingelheim am Rhein(DE)**
Erfinder: **Bechtel,Wolf-Dietrich, Dr.**
**Mühlstrasse 3**
**W-6531 Appenheim(DE)**

## (54) Neue hetrazepinoide Amide.

(57) Die vorliegende Erfindung betrifft neue hetrazepinoide Säureamide Verfahren zu ihrer Herstellung sowie ihre Verwendung als Arzneimittel mit PAF-antagonistischer Wirkung.

EP 0 475 204 A2

Die vorliegende Erfindung betrifft neue hetrazepinoide Amide, Verfahren zu ihrer Herstellung sowie ihre Verwendung als Arzneimittel mit PAF-antagonistischer Wirkung.

Substituierte Thienodiazepine mit PAF-antagonistischer Wirkung sind aus zahlreichen Publikationen und Patentanmeldungen bekannt, so z.B. aus den europäischen Patentanmeldungen 176 927, 194 416, 254 245, 230 942. 341 558. 341 559, 368 175 sowie aus der PCT-Anmeldung 89/0922 bekannt.

Überraschenderweise wurde gefunden, daß Säureamide definierter Struktur verbesserte pharmakologische Eigenschaften aufweisen.

Die neuen Heterazepine entsprechen der allgemeinen Formel I

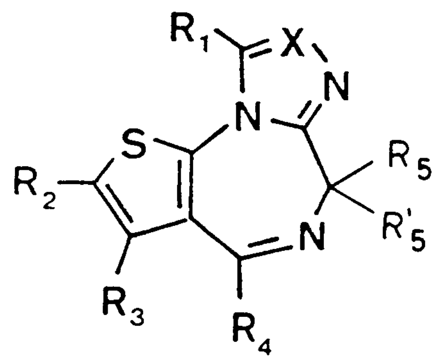

worin

$R_1$ Wasserstoff, Halogen, eine verzweigte oder unverzweigte Alkylgruppe mit 1 bis 4 Kohlenstoffatom(en), bevorzugt Methyl, die gegebenenfalls durch Hydroxy oder Halogen substituiert sein kann, eine Cyclopropylgruppe, eine Cyclobutylgruppe, eine Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;

$R_2$ den Rest $-Z-R_a$

Z eine verzweigte oder unverzweigte Alkyl-, Alkenyl- oder Alkinylgruppe mit n Kohlenstoffatomen, wobei Z gegebenenfalls zusätzlich durch einen Arylrest oder zusätzlich durch $R_a$ substituiert sein kann;

n eine der Zahlen 0, 1, 2, 3, 4, 5, 6, 7, 8, 9 oder 10, wobei im Falle Z einen Alkenyl- oder Alkinylrest bedeutet, n >1 ist und bevorzugt die Bedeutung 2, 3 und 4 besitzt;

$R_3$ Wasserstoff oder Methyl

oder

$R_2$ und $R_3$ zusammen einen Rest der allgemeinen Formel

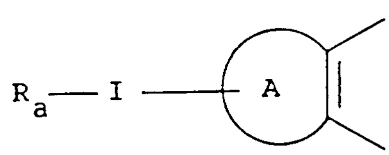

worin

A einen ankondensierten einfach ungesättigten 5-, 6- oder 7-gliedrigen carbocyclischen Ring,

I eine verzweigte oder unverzweigte Alkyl-, Alkenyl- oder Alkinylgruppe mit m Kohlenstoffatomen;

m 0, 1 2, 3, 4, 5 oder 6, bevorzugt O;

$R_a$ einen Rest der allgemeinen Formel

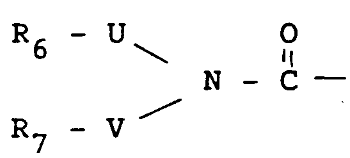

worin

U und V unabhängig voneinander, gleich oder verschieden eine Einfachbindung, eine verzweigte oder unverzweigte $C_1$-$C_6$, bevorzugt $C_1$-$C_3$-Alkylgruppe, eine $C_2$-$C_6$, bevorzugt $C_2$-$C_3$-Alkenylgruppe, oder eine $C_2$ bis $C_6$, bevorzugt $C_3$-$C_4$-Alkinylgruppe bedeuten können,

$R_6$ Wasserstoff oder einen Rest der Formel

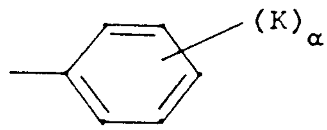

oder

gegebenenfalls substituiertes $C_3$-$C_6$-Cycloalkyl- oder $C_5$-$C_6$-Cycloalkenyl;

$R_7$ einen Rest der Formel

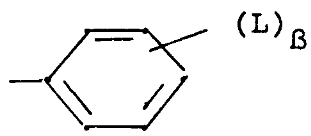

oder

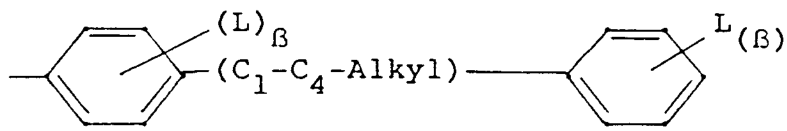

oder

gegebenenfalls substituiertes $C_3$-$C_6$-Cycloalkyl oder $C_5$-$C_6$-Cycloalkenyl; K und L unabhängig voneinander

Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 - Kohlenstoffatom(en), gegebenenfalls substituiertes $C_3$-$C_6$-Cycloalkyl,

gegebenenfalls substituiertes, unverzweigtes oder verzweigtes Alkoxy mit 1 bis 8 bevorzugt 1 bis 4 Kohlenstoffatom(en), einen Rest der Formel

$-(F)_{0-1}-E-(F)_{0-1}-H$, $(F)_{0-1}-NO_2$,

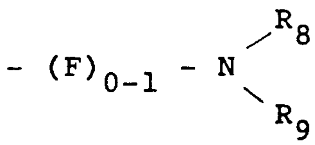 , 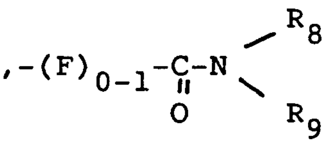 ,

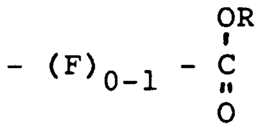

$R = C_1 - C_4$-Alkyl,

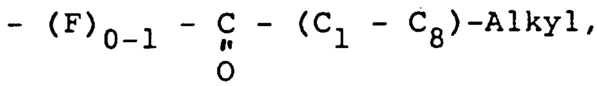

bevorzugt $C_1 - C_4$-Alkyl, worin F eine verzweigte oder unverzweigte Alkylgruppe mit 1 bis 4 Kohlenstoffatom(en), bevorzugt 1 bis 2 Kohlenstoffatom(en)

E = O, S, $N-R_8$, C = O, 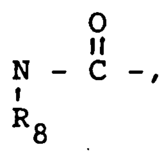 , 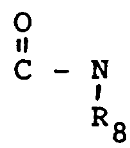

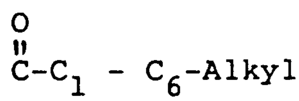 oder 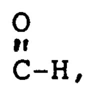

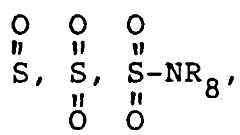

F eine verzweigte oder unverzweigte gegebenenfalls substituierte Alkylgruppe mit 1 bis 8 - bevorzugt 1 bis 4 - Kohlenstoffatom(en), eine gegebenenfalls substituierte Alkenylgruppe mit 2 bis 8 - bevorzugt 2 bis 4 - Kohlenstoffatomen, eine gegebenenfalls substituierte Alkinylgruppe mit 2 bis 8 - bevorzugt 2 bis 4 Kohlenstoffatomen;

worin $R_8$ und $R_9$, die gleich oder verschieden sein können, Wasserstoff, Phenyl, substituiertes Phenyl, eine verzweigte oder unverzweigte Alkyl-, Alkenyl- oder Alkinylgruppe mit 1 bis 6 Kohlenstoffatomen, bevorzugt mit 1 bis 4 Kohlenstoffatom(en), die gegebenenfalls durch Halogen, Hydroxy, Nitro, Amino, substituiertes Amino, $C_3$-$C_6$-Cycloalkyl, bevorzugt Cyclopropyl, $C_1$-$C_4$-Alkoxy, bevorzugt Methoxy;

$R_8$ oder $R_9$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring, einen gegebenenfalls substituierten $C_3$- bis $C_7$-Cycloalkylrest, einen gegebenenfalls substituierten Cycloalkenylrest;

oder

$R_8$ und $R_9$, sofern strukturell möglich, zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome

Stickstoff, Sauerstoff oder Schwefel enthalten kann, wobei jedes weitere Stickstoffatom durch eine verzweigte oder unverzweigte Alkylgruppe mit 1 bis 4 Kohlenstoffatom(en), bevorzugt Methyl, substituiert sein kann;
$\alpha$ und $\beta$ unabhängig voneinander eine der Zahlen 1, 2, 3, 4 oder 5, wobei bei $\alpha$ oder $\beta$ größer 1 alle K und L gleich, teilweise gleich oder verschieden sein können;

$R_6$ ein C-verknüpfter gesättigter oder ungesättigter 5- oder 6-gliedriger Heterocyclus, der als Heteroatome Stickstoff, Sauerstoff und/oder Schwefel enthalten kann - bevorzugt ein Pyridyl-, Thiophen- oder Furanrest, der gegebenenfalls ein- oder mehrfach, gleich oder verschieden durch Halogen,
$C_1$-$C_4$-Alkyl, $C_1$-$C_4$-Halogenalkyl, bevorzugt $CF_3$, Nitro, $NR_8R_9$, S-$C_1$-$C_4$-Alkyl substituiert sein kann,

$R_7$ ein C-verknüpfter gesättigter oder ungesättigter 5- oder 6-gliedriger Heterocyclus, der als Heteroatome Stickstoff, Sauerstoff und/oder Schwefel enthalten kann - bevorzugt ein Pyridyl-, Thiophen- oder Furanrest, der gegebenenfalls ein- oder mehrfach, gleich oder verschieden durch Halogen, $C_1$-$C_4$-Alkyl, $C_1$-$C_4$-Halogenalkyl, bevorzugt $CF_3$, Nitro, $NR_8R_9$, S-$C_1$-$C_4$-Alkyl substituiert sein kann,

$R_4$ Phenyl, wobei der Phenylring ein- oder mehrfach, bevorzugt in 2-Stellung, durch Methyl, bevorzugt Halogen, besonders bevorzugt Chlor oder Brom, Nitro, Alkoxy, bevorzugt Methoxy und/oder Trifluormethyl substituiert sein kann, Pyridyl oder Thienyl, das gegebenenfalls durch $C_1$-$C_4$-Alkyl oder Halogen substituiert sein kann;

$R_5$ Wasserstoff, Methyl, Trifluormethyl, Cyclopropyl, Hydroxymethyl;

$R'_5$ Wasserstoff, Methyl;

X Stickstoff, C-H, C-$CH_3$;

bedeuten können,
gegebenenfalls in Form ihrer Racemate, ihrer Enantiomere, ihrer Diastereomere und ihrer Gemische sowie gegebenenfalls ihrer physiologisch unbedenklichen Säureadditionssalze. [Kohlenstoffatome, die ein Asymmetriezentrum verkörpern, sind im folgenden - ggf. - mit einem Stern gekennzeichnet. In den Beispielen sind Racemate mit dem Präfix (+/-) gekennzeichnet].
Bevorzugt sind Verbindungen der allgemeinen Formel Ia

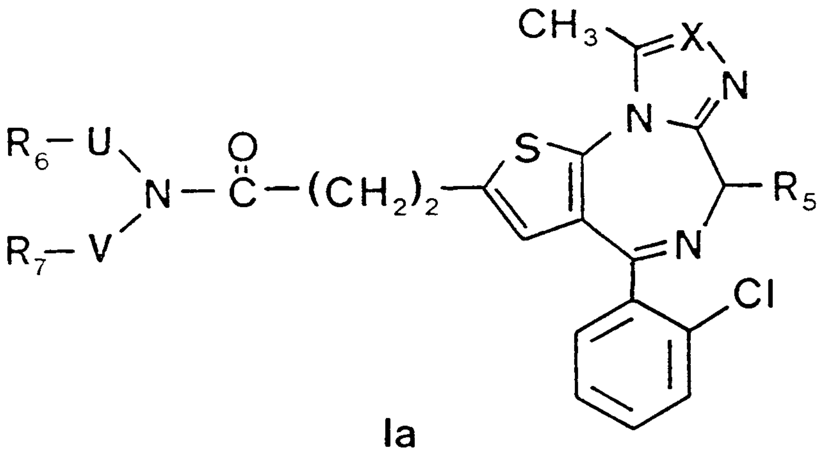

Ia

worin

U eine Einfachbindung, verzweigtes oder unverzweigtes $C_1$-$C_4$-Alkyl, $C_3$ oder $C_4$ Alkenyl, $C_3$ oder $C_4$-Alkinyl;

V eine Einfachbindung, verzweigtes oder unverzweigtes $C_1$-$C_4$-Alkyl;

$R_5$ Wasserstoff oder Methyl;

$R_6$ einen Rest der Formel

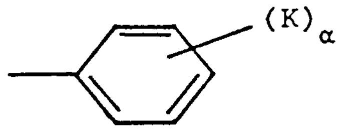

wie zuvor definiert, Cyclopropyl, Cyclopentyl, Cyclohexyl, oder bevorzugt Wasserstoff;

$R_7$ einen Rest der Formel

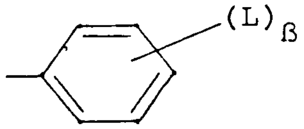

/

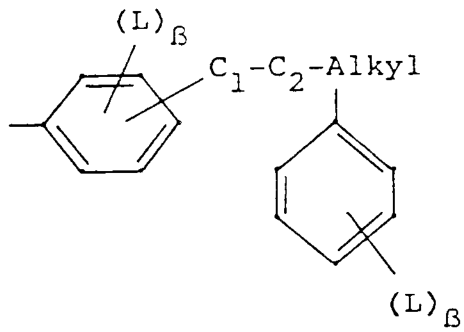

wie zuvor definiert, ein Pyridinyl-, ein Thien-2-yl, oder ein Furan-2-ylrest und

X Stickstoff oder CH bedeuten können.

Bevorzugte Verbindungen in Form ihrer Racemate, besonders bevorzugt in ihren optisch aktiven Isomeren der allgemeinen Formel I mit einem ankondensierten carbocyclischen Ring sind

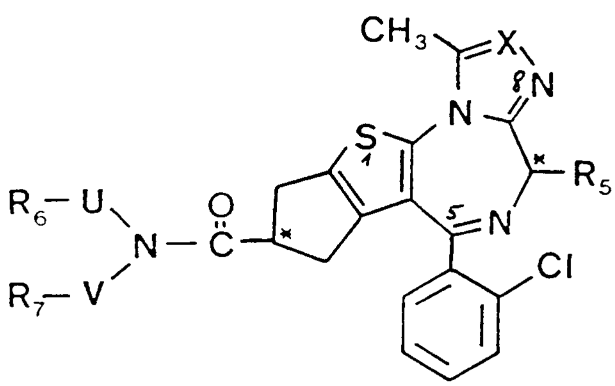

**Ib**

und

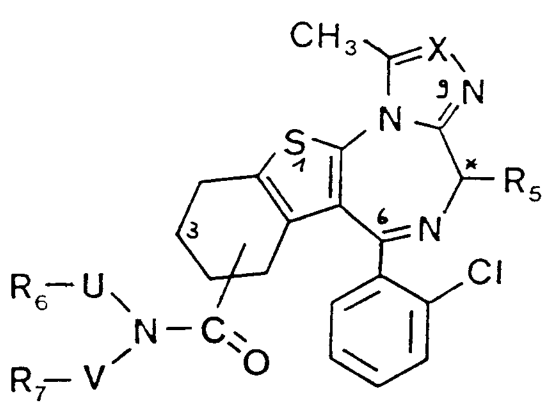

**Ic**

worin

U eine Einfachbindung, verzweigtes oder unverzweigtes $C_1$-$C_4$-Alkyl, $C_3$ oder $C_4$ Alkenyl, $C_3$ oder $C_4$-Alkinyl;

V eine Einfachbindung, verzweigtes oder unverzweigtes $C_1$-$C_4$-Alkyl

$R_5$ Wasserstoff oder Methyl;

$R_6$ einen Rest der Formel

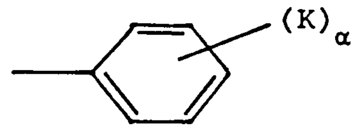

wie vor definiert, Cyclopropyl, Cyclopentyl, Cyclohexyl, bevorzugt Wasserstoff;

$R_7$ einen Rest der Formel

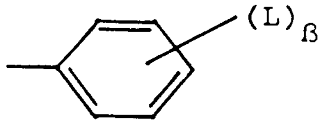
,
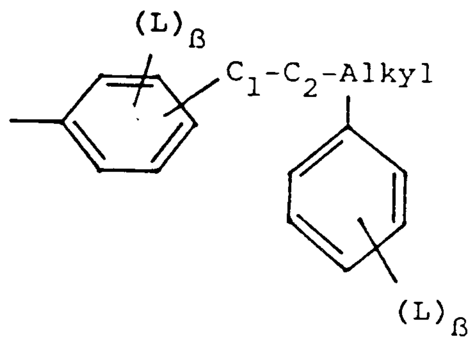

wie zuvor definiert, ein Pyridinyl-, ein Thien-2-yl- oder ein Furan-2-ylrest und

X Stickstoff oder CH bedeuten können.

Besonders bevorzugt sind Verbindungen der allgemeinen Formel Ia, Ib und Ic, worin X Stickstoff bedeutet,

U bevorzugt eine Einfachbindung oder $C_1$-$C_4$-Alkyl, bevorzugt Propyl, Allyl, Propargyl;

V $C_1$-$C_4$-Alkyl, bevorzugt Methylen;

$R_6$ bevorzugt Wasserstoff, Cyclopropyl oder

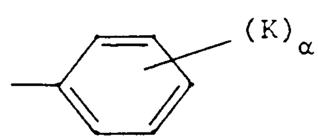

$R_7$

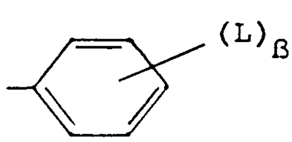
,
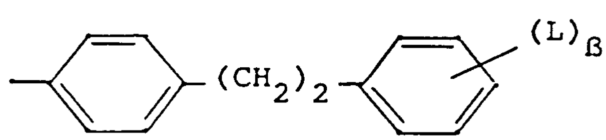

K und L unabhängig voneinander Wasserstoff, gegebenenfalls durch Halogen, Hydroxy, substituiertes $C_1$-$C_4$-Alkyl, $C_2$-$C_4$-Alkinyl, $C_2$-$C_4$-Alkenyl, Nitro, Cyano, Hydroxy, $C_1$-$C_4$-Alkoxy, $C_1$-$C_4$-Alkoxy-$C_1$-$C_4$-alkyl, $C_1$-$C_4$-Alkylsulfonyl, Halogen, $C_1$-$C_4$-Hydroxyalkyl-sulfonyl, $COOC_1$-$C_4$-Alkyl,

$COOC_1$-$C_4$-Alkylphenyl, Cyclopropyl, SH, S-$C_1$-$C_4$-Alkyl;
$\alpha$ 1, 2 oder 3, bevorzugt 1;
$\beta$ 1, 2 oder 3, bevorzugt 1;
bevorzugt
$(K)_\alpha$ Wasserstoff, $CF_3$ oder Halogen
und
$(L)_\beta$ bevorzugt Wasserstoff, F, Cl, Br, Methyl, Trifluormethyl, $CH_3O_2S$, Hydroxy, wobei $(K)_\alpha$ und $(L)_\beta$ bevorzugt in der 4-Position des Phenylringes angeordnet ist.

Besonders bevorzugt sind ebenfalls die S(-)-Isomeren der allgemeinen Formel Ib für den Fall, daß $R_5$ Wasserstoff ist.

Die bei der Synthese gegebenenfalls anfallenden Gemische optisch isomerer Verbindungen können durch Bildung von Diastereomeren und nachfolgender an sich bekannte Verfahren, wie z.B. durch Kristallisation, durch chromatographische oder enzymatische Trennverfahren in die einzelnen optischen Isomeren getrennt werden.

Gegenstand der Erfindung sind die einzelnen Isomeren, deren Mischungen sowie gegebenenfalls die entsprechenden physiologisch geeigneten Säureadditionssalze mit anorganischen oder organischen Säuren. Bevorzugt sind bespielsweise Salze mit Chlorwasserstoffsäure, Bromwasserstoffsäure, Schwefelsäure, Phosphorsäure, Methansulfonsäure, Ethansulfonsäure Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Soweit nicht im einzelnen abweichende Angaben gemacht werden, werden die allgemeinen Definitionen im folgenden Sinn gebraucht:

Alkyl steht im allgemeinen für einen unverzweigten oder verzweigten Kohlenwasserstoffrest mit 1 bis 6 Kohlenstoffatom(en), der gegebenenfalls mit einem Halogenatom oder mehreren Halogenatomen - vorzugsweise Fluor - substituiert sein kann, die untereinander gleich oder verschieden sein können, wobei Niederalkylreste für einen verzweigten oder unverzweigten Kohlenwasserstoffrest mit 1 bis etwa 4 Kohlenstoffatom(en) bevorzugt sind.

Als Alkylgruppen (auch soweit sie Bestandteile anderer Reste sind) werden bevorzugt, soweit nichts anderes angegeben ist, Methyl, Ethyl, Propyl, iso-Propyl, n-Propyl, n-Butyl, iso-Butyl, sek-Butyl und tert.-Butyl.

Alkenyl steht im allgemeinen für einen geradkettigen oder verzweigten Kohlenwasserstoffrest mit 2 bis 6 Kohlenstoffatomen und mit einer oder mehreren, bevorzugt mit einer Doppelbindung, der gegebenenfalls mit einem Halogenatom oder mehreren Halogenatomen - vorzugsweise Fluor - substituiert sein kann, die untereinander gleich oder verschieden sein können. Bevorzugt ist ein Niederalkenylrest mit 2 bis etwa 4 Kohlenstoffatomen und einer Doppelbindung.

Als Beispiele seien Vinyl, Allyl, Propenyl, Isopropenyl, Pentenyl und Isopentenyl genannt.

Alkinyl steht im allgemeinen für einen geradkettigen oder verzweigten Kohlenwasserstoffrest mit 2 bis 6 Kohlenstoffatomen und mit einer oder mehreren, bevorzugt mit einer Dreifachbindung, der gegebenefalls substituiert sein kann.

Bevorzugt ist ein Niederalkinylrest mit 2 bis 4 Kohlenstoffatomen und einer oder zwei Dreifachbindung-(en), der gegebenenfalls mit einem Halogenatom oder mehreren Halogenatomen substituiert sein kann, die untereinander gleich oder verschieden sein können. Als Beispiele seien Ethinyl, Propargyl und 2-Butinyl genannt.

Cycloalkyl steht im allgemeinen für einen gesättigten oder ungesättigten cyclischen Kohlenwasserstoffrest mit 3 bis 6 Kohlenstoffatomen, der gegebenenfalls mit einem Halogenatom oder mehreren Halogenatomen einer Hydroxygruppe, einer Alkylgruppe, bevorzugt Methyl substituiert sein kann, die untereinander gleich oder verschieden sein können. Als Beispiele seien Cyclopropyl, Cyclobutyl, Cyclopentyl, Cyclopentenyl, Cyclohexyl, Cyclohexenyl genannt.

Phenylreste können beispielsweise mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden - substituiert sein.

Eine substituierte Phenylgruppe kann beispielsweise auch einen oder mehrere der nachfolgend genannten Substituenten tragen: $C_1$-$C_6$-Alkyl, $C_1$-$C_6$-Alkoxy, Halogen, Amino, Alkylamino, Dialkylamino, $CF_3$, $C_3$-$C_6$-Cycloalkyl, Cyano, $NO_2$, COH, COOH, $COOC_1$-$C_4$-Alkyl, Cyclopropyl, Hydroxy, SH, S-$C_1$-$C_4$-Alkyl, Hydroxymethyl.

Beispielhaft für substituiertes Phenyl werden genannt: 3-Chlorphenyl, 4-Chlorphenyl, 3-Bromphenyl, 4-Bromphenyl, 4-Fluorphenyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert-Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl.

Alkoxy steht im allgemeinen für einen über ein Sauerstoffatom gebundenen geradkettigen oder verzweigten Kohlenwasserstoffrest mit 1 bis 18 Kohlenstoffatomen. Bevorzugt ist Niederalkoxyrest mit 1 bis etwa 6 Kohlenstoffatomen. Besonders bevorzugt ist ein Alkoxyrest mit 1 bis 4 Kohlenstoffatomen. Als Beispiele seien Methoxy, Ethoxy, Propoxy, Isopropoxy, Butoxy, Isobutoxy, tert.-Butoxy, Pentoxy, Isopentoxy, Hexoxy, Isohexoxy, Heptoxy, Isoheptoxy, Octoxy oder Isooctoxy genannt.

Die Angabe der Zahl der Kohlenstoffatome bezieht sich, sofern nichts anderes angegeben, auf die Länge der Alkyl-, Alkenyl- oder Alkinylkette.

Besonders bevorzugt sind anellierte 6-gliedrige Ringe A, worin die Seitenkette -I-$R_a$ in der 3- oder 4-Position des Hetrazepins oder anellierte 5-gliedrige Ringe A, worin die Seitenkette -I-$R_a$ in der 3-Position des Hetrazepins substituiert ist.

Als Heterocyclus im Rahmen der oben angegebenen Definition steht im allgemeinen für einen 5- bis 7-gliedrigen Ring, der als Heteroatome Sauerstoff, Schwefel und/oder Stickstoff enthalten kann und an den ein weiterer aromatischer Ring bevorzugt ein Phenylring ankondensiert sein kann. Bevorzugt sind 5- und 6-gliedrige aromatische Ringe, die einen Sauerstoff, einen Schwefel und/oder bis zu zwei Stickstoffatomen enthalten, beispielsweise Thienyl, Furyl, Pyridyl, Pyrimidyl, Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl.

Der Heterocyclus kann durch Halogen, Hydroxy und/oder verzweigtes oder unverzweigtes Alkyl mit 1 bis 4 Kohlenstoffatom(en) substituiert sein.

Beispiele für gegebenenfalls substituierte gesättigte oder ungesättigte heterocyclische 5-, 6- oder 7-gliedrige Ringe bzw. Heteroarylreste sind:

Pyrrol, Pyrrolin, Pyrrolidin, 2-Methylpyrrolidin, 3-Methylpyrrolidin, Piperidin - gegebenenfalls durch $C_1$-$C_4$ Alkyl ein- oder mehrfach substituiert - Piperazin, N-Methylpiperazin, N-Ethylpiperazin, N-n-Propylpiperazin, N-Benzylpiperazin, Morpholin, Thiomorpholin, Imidazol, Imidazolin, Imidazolidin, Triazol, Pyrazol, Pyrazolin, Pyrazolidin, Triazin, 1, 2, 3, 4 - Tetrazin, 1, 2, 3, 5 - Tetrazin, 1, 2, 4, 5 - Tetrazin - wobei die genannten Heterocyclen durch Alkyl mit 1 bis 4 Kohlenstoffatomen - bevorzugt Methyl - substituiert sein können;

Als heterocyclische Reste, die über ein Kohlenstoffatom verknüpft sein können, werden beispielsweise Thiophen, 2-Methylthiophen, Furan, Tetrahydrofuran, 2-Methyltetrahydrofuran, 2-Hydroxymethylfuran, $\alpha$-Pyran, $\gamma$-Pyran, 1,3-Dioxolan, 1,2-Oxathiolan, 1,2-Oxathiepan, Tetrahydro-pyran, Thiolan, 1,3-Dithian, 1,3-Dithiolan, 1,3-Dithiolen, genannt, wobei der Heterocyclus durch $C_1$-$C_4$ Alkyl, $C_1$-$C_4$ Alkoxy, Halogen substituiert sein kann.

Bekanntlich handelt es sich bei PAF (Plättchen Aktivierender Faktor) um das Phospholipid Acetyl-glyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.

Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen der allgemeinen Formel I zur Behandlung bei den folgenden Indikationsstellungen:

Obstruktive Lungenerkrankungen, wie z.B. bronchiale Hyperreaktivität, entzündliche Lungenwegserkrankungen, wie z.B. chronische Bronchitis; allergische Rhinitis;

Herz- Kreislauferkrankungen, wie z.B. Polytrauma, Anaphylaxe, Arteriosklerose, entzündliche Darmerkrankungen, EPH-Gestose (ederma-proteinuria Hypertension), Erkrankungen des extrakorporalen Kreislaufs, ischämische Erkrankungen, entzündliche und immunologische Erkrankungen, Immunmodulation bei Transplantationen von Fremdgeweben, Immunmodulation bei Leukämie, Metastasenausbreitung z.B. bei bronchialer Neoplasie, Erkrankungen des ZNS, wie z.B. Migräne, Agoraphobie (panic disorder), weiterhin erweisen sich die erfindungsgemäßen Verbindungen als Cyto- und Organoprotektiv, z.B. zur Neuroprotektion, z.B. bei Leberzirrhose, DIC (disiminierte intravasale Gerinnung); Nebenwirkungen einer Arzneimitteltherapie, z.B. anaphylaktoide Kreislaufreaktionen, Kontrastmittelzwischenfälle, Nebenwirkungen bei der Tumortherapie; haemolytik uremic syndrome; Unverträglichkeiten bei Bluttransfusionen; fulminantes Leberversagen ($CCl_4$-Intoxikation) Amanitaphalloides-Intoxikation (Knollenblätterpilzvergiftung); primary biliary, cirrhosis, Symptome von parasitären Erkrankungen (z.B. Wurmerkrankungen); Autoimmunerkrankungen.
Weiterhin sind folgende Indikationen von Interesse: Immunfunktion bei Aids, Diabetes, juvenile Diabetes, diabetische Retinopathie, polytraumatischer Schock, hämorrhagischer Schock, CNS: Ischämie, Multiple Sklerose, Migräne, Colitis ulcerosa, Morbus Crohn, Psoriasis, pulmonaler Hochdruck sowie chronische ischämische Herzinsuffizienz, PAF Antagonisten der allgemeinen Formel I eignen sich zur Behandlung von pahtologischen Blutgasveränderungen, wie beispielsweise respiratorische Acidose, metabolische Alkalose. In Kombination mit Anticholinergica können PAF-Antagonisten zur Verbesserung der Blutgaswerte bei Phosphorsäureestervergiftungen eingesetzt werden. Es ist bekannt, daß PAF-Antagonisten allein - oder in Kombination mit immunsuppressiv wirkenden Verbindungen (z.B. Cyclosporinen) zur Behandlung von Autoimmunerkrankungen und bei Transplantationen eingesetzt werden können.
Weiterhin wird vorgeschlagen, PAF-Antagonisten in Kombination mit Antihystaminica zu verwenden. Bezüglich der Definition der Antihistaminica wird inhaltlich auf die Europäische Patentanmeldung 345 731 und 35 749 Bezug genommen. Von besonderem Interesse sind Kombinationen mit Epinastin, insbesondere von Epinastin und S(-)-3-((4-Trifluormethylbenzyl)propylaminocarbonyl)-5-(2-chlorphenyl)-10-methyl-7H-cyclopenta [4,5]thieno-[3,2-f][1,2,4]-triazolo-[4,3-a][1,4]diazepin. Ferner ist bekannt, daß PAF-Antagonisten in Kombination mit $\beta_2$-Mimetica zur Behandlung des Asthma bronchiale eingesetzt werden können. Auch die Kombination von PAF-Antagonisten mit TNF sind vorteilhaft.
PAF assoziierte Interaktion mit Gewebshormon (autocoid hormones), Lymphokine und anderen Mediatoren. Von Interesse sind beispielsweise auch Kombinationen von Verbindungen der allgemeinen Formel I mit Prostacyclinen, z.B. zur Konservierung von Organen vor Transplantationen. Prostacyclin ist ein natürlich vorkommender Plättchenaggregationsinhibitor, wird jedoch im Körper sehr schnell metabolisiert. Aus diesem Grund wurden bereits stabile Deriate bzw. Analoga des Prostacyclins synthetisiert und in klinischen Prüfungen auf ihre Eignung hinsichtlich der Substitution von Protacyclin untersucht. Hierzu zählen beispielsweise Ataprost,, Beraprost, Cicaprost, Ciprosten, Eptalprost, Iloprost, Nileprost, 7-Oxo-prostaglandine $I_2$, Taprosten, TEI-8166 und TRY-2000.

Die neuen Hetrazepine sind sehr starke PAF-Antagonisten und anderen bekannten diazepinoiden PAF-Antagonisten in folgenden Kriterien überlegen:

- es besteht eine totale Dissoziation zwischen dem PAF-Antagonismus und den Benzodiazepin-Receptor vermittelten Effekten;
- sie zeigen eine überlegene Bindungsaffinität zum PAF-Rezeptor auf gewaschenen Humanplättchen und zeigen eine stärkere Hemmung der PAF-induzierten Plättchenaggregation;
- Darüber hinaus hemmen sie in einer überlegenen Weise die PAF-(30 ng/kg x min) induzierte Bronchokonstriktion nach oraler und parenteraler Applikation am Meerschweinchen in Kombination mit einer sehr langen Wirkdauer (länger als 15 Std. nach oraler Applikation am Meerschweinchen).

Die nachfolgende Tabelle zeigt einige ausgewählte Verbindungen mit den dazugehörigen Werten zur Hemmung der PAF-induzierten Plättchenaggregation.

| Beispiel | PAF-induzierte Thrombozythenaggregation [µmol] |
|---|---|
| 2 | 0.049 |
| 6 | 0.023 |
| 7 | 0.084 |
| 10 | 0.037 |

Methodik

Gesunden männlichen und weiblichen Spendern im Alter von 18 bis 35 Jahren, die mehrere Tage vor

Blutentnahme keine Medikamente (Aspirin oder andere nicht steroidale Entzündungshemmer) eingenommen hatten, wurden jeweils 200 ml Blut aus einer nicht gestauten Vene mit Hilfe einer Plastikspritze, in der sich 3.8 %-ige Natriumcitratlösung befand, entnommen. Das Verhältnis Natriumcitratlösung: Blut betrug 1:9. Das Citratblut wurde in Plastikröhrchen bei 150 x g ( = 1200 U/min) und Raumtemperatur 20 min lang zentrifugiert (Heraeus Christ Tischzentrifuge 124).

Die Messung der Thrombozytenaggregation in vitro erfolgte nach der Methode von Born und Cross (1963), wobei dem TRP unter ständigem Rühren ein Aggregationsauslöser (PAF) zugesetzt wurde. Zur Messung wurde in 1 ml Plastikküvetten, die jeweils einen kleinen Metallstift (Rührer, 1000 U/min) enthielten, 0.8 ml TRP und 0.2 ml modifizierte Tyrode-Lösung (s. unten) gegeben. Die Prüfsubstanz wurde 2 bis 3 min vor Auslösung der Aggregation in einem Volumen von 10 µl zugesetzt. Als Lösungsmittel dienten entweder DMSO und Wasser oder eine verdünnte HCl-Lösung. Die Kontrollansätze enthielten das entsprechende Volumen dieser Lösungsmittel. Nach Registrierung der Ausgangsabsorption (2-3 min) wurde die Aggregation induziert. In einem Volumen von 10 µl erfolgte die Gabe von PAF ($5 \times 10^{-8}$ M; Bachem Feinchemikalien) in die Küvette.

Die modifizierte Tyrode-Lösung hatte folgende Zusammensetzung: 136.9 mM NaCl; 2.68 mM KCl; 0.5 mM $MgCl_2$; 1.8 mM $CaCl_2$; 0.42 mM $NaH_2PO_4$; 5.55 mM Glucose und 11.9 mM $NaHCO_3$.

Zur Beurteilung von Substanzeffekten wurde das Maximum der ersten Aggregationswelle verwendet. Die durch den Aggregationsauslöser induzierte maximale Absorption (= maximale Aggregation = 100 %) wurde gleichzeitig in einem Parallelansatz (im 2. Kanal des Aggregometers) zu jedem Testansatz mitgeführt und als 100 % Wert verwendet. Der unter der Wirkung der Testsubstanz erreichte Aggregationswert wurde als % des Kontrollwertes (-ansatz) angegeben. Mit Hilfe dieses Verfahrens wurden Konzentrationswirkungskurven mit einem Stichprobenumfang von jeweils n = 4 erstellt und $IK_{50}$-Werte (Konzentration bei einer 50 %igen Aggregationshemmung) berechnet.

Die neuen Verbindungen können in üblicher Weise aus den entsprechenden Carbonsäuren der allgemeinen Formel

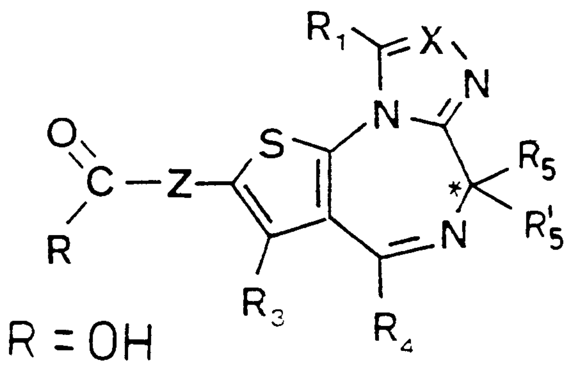

R = OH

IIa

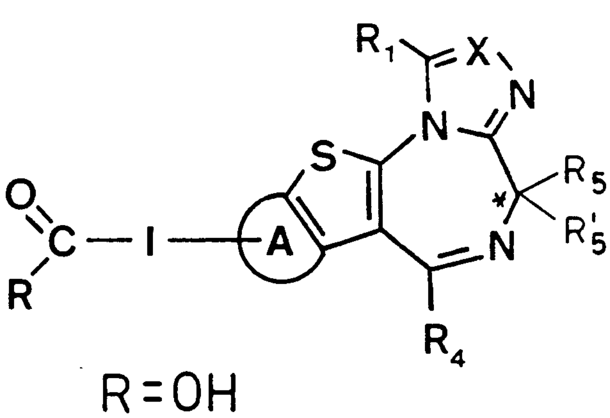

R = OH

IIb

worin die Reste R die einfangs definierte Bedeutung besitzen, erhalten werden, z.B.

a) durch Umsetzung mit einem entsprechenden Amin in Gegenwart eines Carbodiimids oder von Carbonyldiimidazol,

b) durch Überführung der freien Säure in ein Säurehalogenid oder Säureanhydrid und anschließende Umsetzung mit einem entsprechenden Amin.

Die Umsetzung der freien Säure mit dem Amin erfolgt in Gegenwart eines Carbodiimids, beispielsweise von Cyclohexylcarbodiimid, gegebenenfalls unter Zuhilfenahme von Hydroxybenztriazol oder mit Hilfe von Carbonyldiimidazol in einem inerten Lösungsmittel wie Dimethylformamid, Tetrahydrofuran, Dioxan usw. bei Temperaturen zwischen 0 ° C und dem Siedepunkt des Reaktionsgemisches.

Bei der Reaktion des Amins mit einem Säurehalogenid oder Säureanhydrid wird das Amin in einem inerten Lösungsmittel, beispielsweise Dimethylformamid, Tetrahydrofuran, Dioxan oder einem geeigneten Kohlenwasserstoff wie Benzol oder Toluol bei Temperaturen zwischen Raumtemperatur und dem Siedepunkt des Reaktionsgemisches mit dem Säurehalogenid oder dem Säureanhydrid umgesetzt, wobei gegebenenfalls ein säurebindendes Mittel wie Natriumcarbonat, Natriumbicarbonat oder eine tertiäre organische Base, beispielsweise Pyridin oder Triethylamin, zugegeben wird.

Säurehalogenid bzw. Säureanhydrid erhält man aus der freien Säure in üblicher Weise, z.B. durch Reaktion der Säure mit einem Thionylhalogenid oder Phosphoroxyhalogenid bzw. durch Umsetzung eines Alkalisalzes der Säure mit Acetylchlorid oder Chlorameisensäurechlorid.

Die Herstellung der erfindungsgemäßen Verbindungen kann in Analogieverfahren gemäß dem Stand der Technik erfolgen.

In Anlehnung an die Europäische Patentschrift 194 416 und die Europäischen Patentanmeldungen 230 942 und 254 245 erfolgt die Synthese der geeigneten Carbonsäureester der allgemeinen Formel II, R = $COOC_1$-$C_4$-Alkyl, die dann zur Carbonsäure verseift werden können gemäß nachfolgenden Raktionen.

Ausgehend von den entsprechend substituierten Diazepinthionen der allgemeinen Formel IIIa oder IIId - bei denen bei IIIb gegebenenfalls die optische Aktivität am Chiralitätszentrum des carbocyclischen 5- oder 6-Rings gemäß der deutschen Patentanmeldung DE 39 09 012.4 bzw. der korrespondierenden europäischen Patentanmeldung der Anmeldungsnummer 90 104 890.0 oder am Diazepinring mitgebracht werden kann -

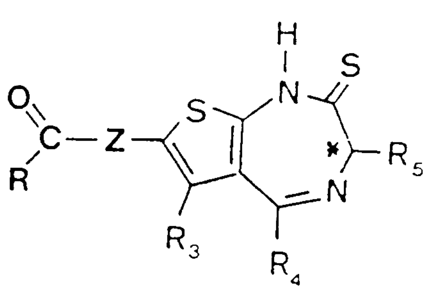

IIIa

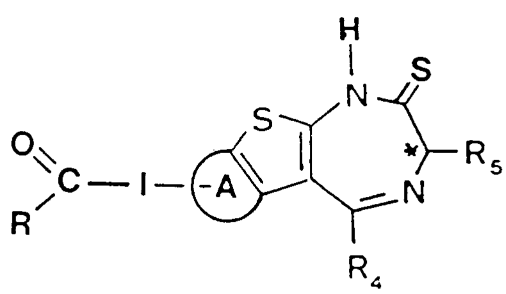

IIIb

worin R, $R_3$, $R_4$, $R_5$, A, I und Z wie zuvor in der Formel II definiert sind, erhält man die Carbonsäureester der allgemeinen Formel IIIa und IIIb (R = Alkyl) dadurch, daß man

A) für den Fall, daß X Stickstoff bedeutet

a) mit einem Säurehydrazid der allgemeinen Formel $R_1$-$CONHNH_2$ umsetzt,

b) oder aber mit Hydrazin in die entsprechende Hydrazono-Verbindung

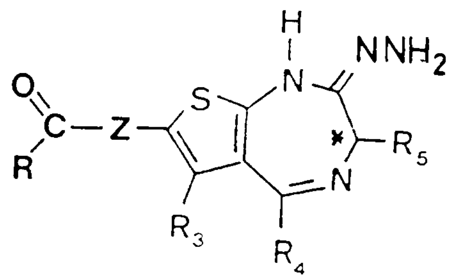

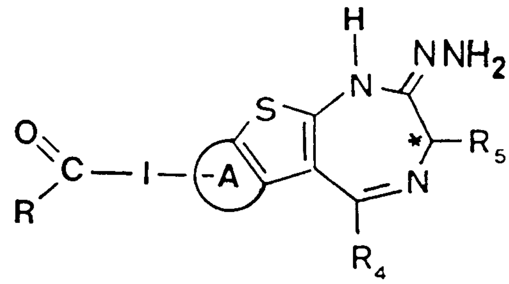

überführt und anschließend mit einem Säurehalogenid der allgemeinen Formel $R_1$-CO-Hal oder mit einem Orthoester der allgemeinen Formel $R_1$-C(OR')$_3$ worin R' eine niedere Alkylgruppe bedeutet, umsetzt, oder

B) Für den Fall, daß X C-H oder C-Alkyl bedeutet

a) mit einem Aminoalkin der allgemeinen Formel

$R'_1$-C≡C-$CH_2$-$NH_2$

worin $R'_1$ Wasserstoff oder eine niedere Alkylgruppe bedeutet

oder aber

b) mit einem α-Aminoaldehyd-alkylacetal oder α-Aminoketon-alkylketal der allgemeinen Formel

$H_2NCH_2$-$CR_1(OR')_2$

worin $R_1$ Wasserstoff oder eine Alkylgruppe mit 1 bis 4 Kohlenstoffatomen und R' eine niedere Alkylgruppe bedeutet, umsetzt.

Anschließend können gewünschtenfalls die Verbindungen des Typs I mit $R_1$ = Wasserstoff in Gegenwart einer Base mit einem Halogenierungsmittel, wie z.B. mit Chlor oder Brom, zu einer Verbindung der allgemeinen Formel I mit $R_1$ Halogen, wie z.B. Chlor oder Brom umgesetzt werden.

Anschließend können gewünschtenfalls die Halogenverbindung in eine Verbindung der allgemeinen Formel I mit $R_1$ = Alkoxy mit 1 bis 4 Kohlenstoffatomen durch Reaktion mit dem entsprechenden Alkoholat überführt werden.

Die Umsetzung des Thions III mit einem Säurehydrazid nach dem Verfahren a) erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen der Raumtemperatur und dem Siedepunkt des Reaktionsgemisches. Die Isolierung der Endprodukte geschieht nach bekannten Methoden, wie z.B. durch Kristallisation.

Die Umsetzung des Thions III mit Hyrazin nach dem Verfahren b) erfolgt in inerten organischen Lösungsmitteln, wie beispielsweise Tetrahydrofuran, Dioxan, halogenierten Kohlenwasserstofen, wie z.B. Methylenchlorid, geeigneten Kohlenwasserstoffen, bei Temperaturen zwischen Raumtemperaturen und dem Siedepunkt des Reaktionsgmisches.

Die dabei entstehenden Hydrazin-1,4-diazepine können nach herkömmlichen Verfahren isoliert oder aber auch direkt weiterverarbeitet werden.

Die weitere Umsetzung mit einem Säurehalogenid oder Orthoester erfolgt in einem inerten organischen Lösungsmittel, wie z.B. halogenierten Kohlenwasserstoffen, cyclischen oder aliphatischen Estern, kann aber auch direkt in Substanz erfolgen. Die Isolierung des Endprodukts Ia erfolgt nach bekannten Methoden, beispielsweise durch Kristallisation.

Falls man das oder die optische Aktivität nicht bereits aus Synthesevorstufen gemäß der deutschen Patentanmeldung DE 39 09 012.4 mitbringt, können die Endverbindungen auch nach an sich bekannten Trennverfahren in ihre Enantiomeren gespalten werden. Dies kann auch auf geeigneten chiralen Säuren geschehen.

Beispiel 1

4-(2-Chlorphenyl)-9-methyl-6H-thieno[3,2-f][1,2,4]triazolo[4,3-a][1,4]diazepino-2-(2-ethyl)-carbonsäurepropyl-

(4-trifluormethylbenzyl)amid

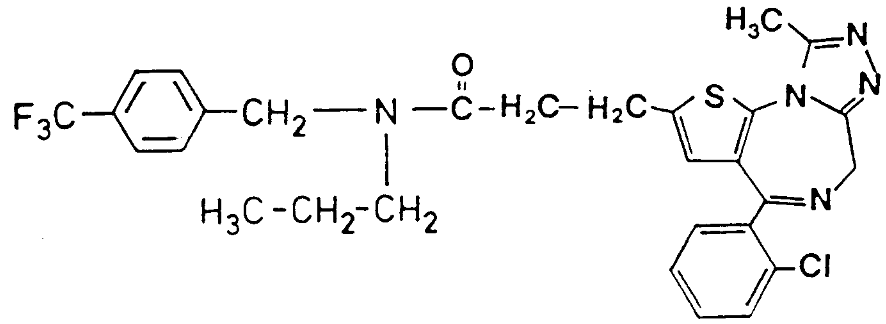

4,6 g (0,068 mol) Imidazol werden in 50 ml Methylenchlorid gelöst und langsam mit 2 g (1,2 ml) Thionylchlorid versetzt. Dazu gibt man während 10-15 Min. bei 15-20 ° C 6,5 g (0,017 mol) 4-(2-Chlorphenyl)-9-methyl-6H-thieno[3,2-f][1,4]-diazepino-2-(2-ethyl)-carbonsäure [W.D. Bechtel und K. H. Weber, J. Pharmac. Sci. 74 (1985) 1265] und läßt 15-20 Min nachrühren. Zu dem sich gebildeten Imidazolid gibt man 3,6 g (0,017 mol) N-propyl-(4-trifluormethylbenzyl)-amin und läßt 1 Stunde nachrühren. Das Reaktionsgemisch wird mit 100 ml Methylenchlorid verdünnt und mit 2 mal 50 ml Wasser ausgeschüttelt. Aus der getrockneten Methylenchloridphase erhält man nach Eindampfen ein Öl, das bei Ätherzugabe kristallisiert.
Ausbeute 5,8 g; Schmp. 182-183 ° C,
$^1$H-NMR ($CDCl_3$):$\delta$ = 7.62-7.14 (8H, m, aryl-H); 6.45, 6,39 (1H, 2s, thiophen-H); 4,92 (2H, s, $CH_2$-7-ring); 4,64, 4,56 (2H, 2s, N-$CH_2$-aryl); 3,42-3,06 (4H, m, $CH_2$-thiophen; N-$CH_2CH_2CH_3$); 2,74, 2,59 (2H, 2t, J = 6Hz, $CH_2$-C = O); 2,69 (3H, s, $CH_3$-triazol); 1,52 (2H, m, N-$CH_2CH_2$-$CH_3$); 0,87 (3H, m, N-$CH_2CH_2CH_3$).

Das N-Propyl-N(4-trifluormethyl)benzylamin wird wie folgt dargestellt:
10 g (0,057 mol) 4-Trifluormethylbenzaldehyd werden unter Eiskühlung und Rühren zu 3,8 g (0,065 mol) Propylamin in 100 ml Ethanol zugetropft. Man läßt 1 Stunde bei Raumtemperatur rühren, kühlt erneut mit Eis und gibt allmählich bei 10-15 ° C 1,4 g (0,036 mol) Natriumborhydrid hinzu. Anschließend wird 30 Minuten bei Raumtemperatur und 1 Stunde bei 35-40 ° C gerührt. Mit 2 n Salzsäure wird ein pH-Wert von 5 eingestellt und der Alkohol im Vakuum abdestilliert. Zu dem Rückstand gibt man verdünnten Ammoniak bis zur deutlich alkalischen Reaktion und schüttelt das Amin mehrmals mit Essigester aus. Nach dem Trocknen und Eindampfen wird der Rückstand im Vakuum destilliert ($Kp_{0.15}$: 63 ° C).
$^1$H-NMR ($CDCl_3$):$\delta$ = 7.49 (4H, m, aryl-H); 3.84 (2H, 2s, N-$CH_2$-aryl); 2.59 (2H, t, J = 6Hz, N-$CH_2CH_2$-$CH_3$); 1.54 (2H, m, N-$CH_2CH_2CH_3$); 1,46 (1H, s, breit, NH); 0,93 (3H, t, J = 6HZ, N-$CH_2CH_2$-$CH_3$).

Beispiel 2

S(-)-3-(Benzylpropylaminocarbonyl)-5-(2-chlorphenyl)-10-methyl-7H-cyclopenta[4,5]thieno[3,2-f][1,2,4]-triazolo[4,3-a][1,4]diazepin

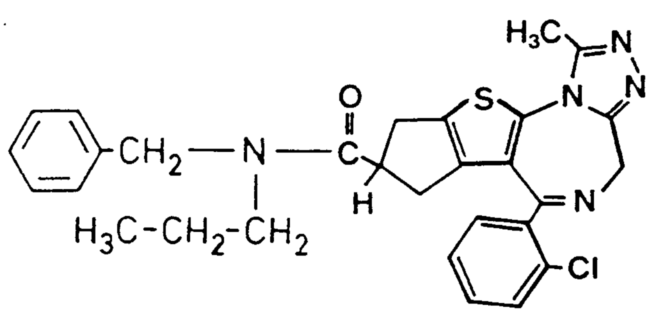

1,5 g (0,0038 mol) S(-)3-Carboxy-5-(2-chlorphenyl)-10-methyl-7H-cyclopenta[4,5]thieno[3,2-f][1,2,4]triazolo-[4,3-a][1,4]diazepin [deutsche Patentanmeldung 39 09 012.4 bzw. europäische Patentanmeldung der Anmeldungsnummer 90 104 890.0] werden in 20 ml Dimethylformamid gelöst bzw. suspendiert und bei 0-10 ° C 0.6 g (0,004 mol) N-Propylbenzylamin, 0.61 g Hydroxybenztriazol und 0.91 g Dicyclohexylcarbodiimid

zugegeben. Man rührt 8 Stunden bei dieser Temperatur, läßt über Nacht im Kühlschrank stehen und saugt anschließend den ausgefallenen Harnstoff ab. Das Filtrat wird mit 50 ml Essigester verdünnt und mit 20 ml Wasser ausgeschüttelt, getrocknet, filtriert und im Vakuum eingedampft. Den Rückstand nimmt man in Methylenchlorid auf, wäscht mit Natriumbicarbonatlösung, trocknet und dampft erneut ein. Nach Filtration über eine $SiO_2$-Säule (Elution Methylenchlorid/Methanol 98:2), Eindampfen des Eluates und Umkristallisation aus Isopropanol erhält man 1.8 g Kristalle vom Fp. 128-130 °C, $[\alpha]_D^{20}$ = -20.9 ° (1 %, Methanol)
Die optische Reinheit der Titelverbindung wird durch HPLC-Chromatographie auf einer chiralen Polyamidsäule bestimmt und beträgt mindestens 99 %.
$^1$H-NMR ($CDCl_3$):δ = 7.45-6,97 (9H, m, aryl-H); 4.90 (2H, s, breit, $CH_2$-7ring), 4,56, 4,59 (2H, 2-AB-Systeme, N-$CH_2$-aryl); 3,88-2,98 (5H, m, N-$CH_2$-$CH_2CH_3$; CH-C = O; thiophen-$CH_2$-2); 2,70; 2,68 (3H, 2s, $CH_3$-triazol); 2,55-1,90 (2H, m, thiophen-$CH_2$-3); 1,52 (2H, m, N-$CH_2CH_2CH_3$).

Beispiel 3

( + /-)-3-(4-(Methoxybenzyl)methylaminocarbonyl)-5-(2-chlorphenyl)-10-methyl-7H-cyclopenta[4,5]thieno[3,2-f][1,2,4]triazolo-[4,3-a][1,4]diazepin

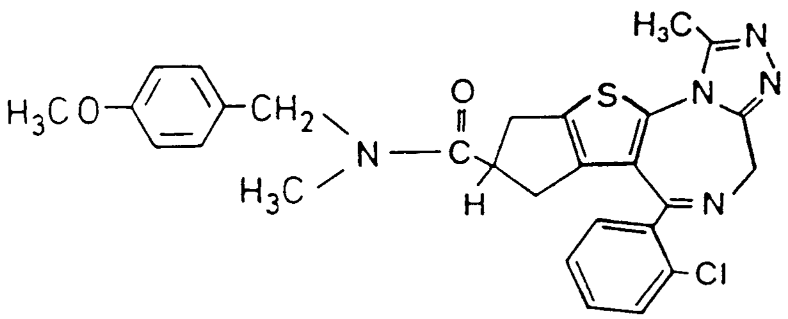

1,5 g (0,0038 mol) racemisches 3-Carboxy-5-(2-chlorphenyl-10-methyl-7H-cyclopenta[4,5]thieno[3,2-f]-[1,2,4]triazolo-[4,3-a][1,4]diazepin werden in 0,5 g Thionylchlorid, das man mit 50 ml verdünnt Methylenchlorid, gegeben und 2 Stunden bei Raumtemperatur gerührt. Unter Eiskühlung fügt man langsam so viel 4-Methoxybenzylmethylamin hinzu, bis ein pH-Wert von 6 erreicht ist. Man rührt 2 Stunden bei Raumtemperatur, schüttelt 2 mal mit je 20 ml $H_2O$, trocknet die organische Phase, destilliert das Lösungsmittel ab und chromatographiert über $SiO_2$. Man erhält 1.6 g der Titelverbindung.
$^1$H-NMR (DMSO-d6): δ = 7.45 - 6.78 ((8H, m, aryl-H); 4.80 (2H, AB-System, $CH_2$-7-ring); 4.39 (2H, s, N-$CH_2$-aryl); 3.87 (1H, m, CH-C = 0); 3.75 (3H, s, $OCH_3$); 3.15 (2H, m, $CH_2$-2); 2.79 (3H, s, N-$CH_3$); 2.59 (3H, s, $CH_3$-triazol); 2.19 (2H, m, $CH_2$-4).

Die nachfolgend aufgeführten Verbindungen können auf analoge Weise erhalten werden:

Beispiel 4

S(-)-3-(Benzylaminocarbonyl)-5-(2-chlorphenyl)-10-methyl-7H-cyclopenta[4,5]thieno-[3,2-f][1,2,4]triazolo-[4,3-a][1,4]diazepin

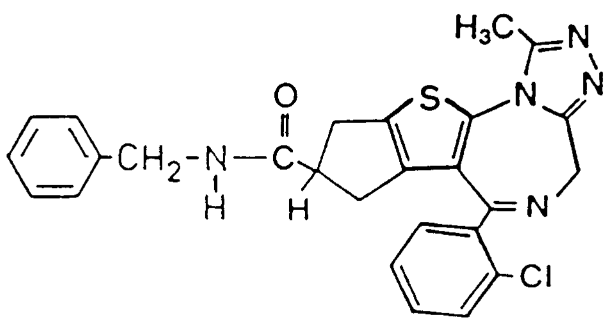

$[\alpha]_D$ = -37.4 (1 %, Methanol)
$^1$H-NMR ($CDCl_3$):$\delta$ = 7.47-7,14 (9H, m, aryl-H); 5.89 (1H, 7, J=5Hz, NH); 4,88 (2H, s, breit, $CH_2$-7-ring), 4,40 (2H, m $CH_2$-aryl); 3.43-3.09 (3H, m, O=C-CH-$CH_2$); 2.68 (3H, s, $CH_3$-triazol); 2.50-2.06c(2H, m, $CH_2$-4-5-ring).

Beispiel 5

S(-)-3-(Benzylmethylaminocarbonyl-5-(2-chlorphenyl)-10-methyl-7H-cyclopenta[4,5]thieno-[3,2-f][1,2,4]-triazolo-[4,3-a][1,4]diazepin

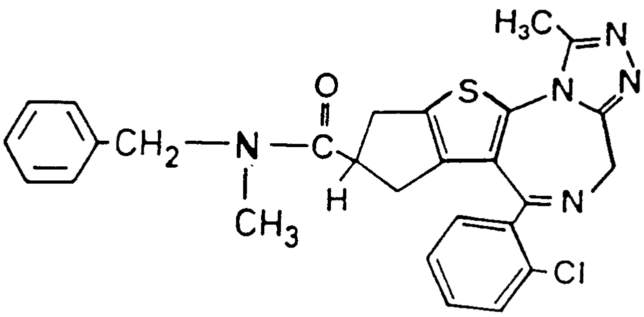

$[\alpha]_D$ = -31.4 (1 %, Methanol); Fp.: 202 - 204 °C.
$^1$H-NMR (DMSO-d6):$\delta$ = 7.47-7.06 (9H, m, aryl-H); 4.80 (2H, AB-system, $CH_2$-7-ring), 4.47 (2H, s, N-$CH_2$); 3.87 (1H, m, CH-C=O); 3,17 (2H, m, $CH_2$-2); 2.83 (3H, s, N-$CH_3$); 2.59 (3H, s, $CH_3$-triazol); 2.20 (2H, m, $CH_2$-4).

Beispiel 6

S(-)-3-(Benzylethylaminocarbonyl)-5-(2-chlorphenyl)-10-methyl-7H-cyclopenta[4,5]thieno-[3,2-f][1,2,4]-triazolo-[4,3-a][1,4]diazepin

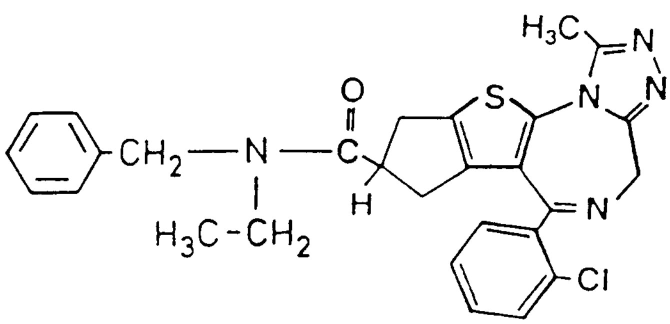

$[\alpha]_D$ = -27.6 ° (1 %, Methanol), Fp.: 204 - 205 °C.
$^1$H-NMR ($CDCl_3$):$\delta$ = 7.47-7.00 (9H, m, aryl-H); 4.89 (2H, s, breit, $CH_2$-7-ring), 4.56; 4.47 (2H, 2 AB-system, $NCH_2$-aryl); 3.87-2.93 (5H, m, N-$CH_2$-$CH_3$, NCOCH-$CH_2$(2)); 2.70; 2.68 (3H, 2s, $CH_3$-triazol); 2.57-2.00 (2H, m, $CH_2$(4); 1.07 (3H, t, J=6Hz, N-$CH_2$-$CH_3$).

Beispiel 7

S(-)-3-(Benzylbutylaminocarbonyl)-5-(2-chlorphenyl)-10-methyl-7H-cyclopenta[4,5]thieno-[3,2-f][1,2,4]-triazolo-[4,3-a][1,4]diazepin

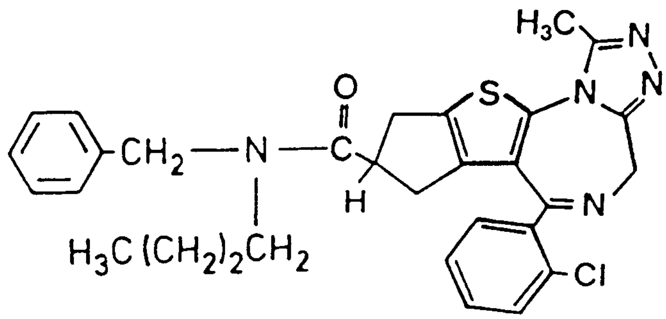

$[\alpha]_D$ = -24.5° (1 %, Methanol)
$^1$H-NMR ($CDCl_3$):$\delta$ = 7.47-7.01 (9H, m, aryl-H); 4.89 (2H, s, breit, $CH_2$-7-ring), 4.56; 4.49 (2H, 2 AB-system, N-$CH_2$-aryl); 3.88-2.93 (5H, m, N-$CH_2$($CH_2$(2)-$CH_3$); O=C-CH-$CH_2$(2)); 2.70; 2.68 (3H, 2s, $CH_3$-triazol); 2.54-2.01 (2H, m, $CH_2$(4); 1.60-1.13 (4H, m, N-$CH_2CH_2CH_2CH_3$); 0.88 (3H, t, J=7Hz, N-$(CH_2)_3$-$CH_3$).

Beispiel 8

S(-)-3-(Benzylisopropylaminocarbonyl)-5-(2-chlorphenyl)-10-methyl-7H-cyclopenta[4,5]thieno-[3,2-f][1,2,4]-triazolo-[4,3-a][1,4]diazepin

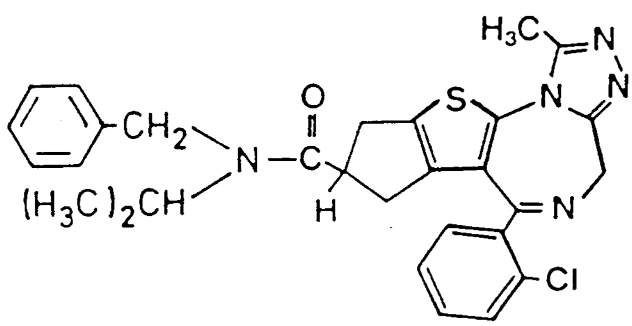

$[\alpha]_D$ = -17.5° (1 %, Methanol)
$^1$H-NMR ($CDCl_3$):$\delta$ = 7.47-7.06 (9H, m, aryl-H); 4.87 (2H, s, breit, $CH_2$-7-ring), 4.82 (1H, qu, J=6Hz, N-CH); 4.47; 4.40 (2H, 2-AB-system, N-$CH_2$-aryl); 4.11-2.93 (3H, m, O=C-CH-$CH_2$(2)); 2.69; 2.67 (3H, 2s, $CH_3$-triazol); 2.50-1.79 (2H, m, $CH_2$(4)); 1.10 (4H, m, ($CH_3$(2)-CH-).

Beispiel 9

S(-)-3-(Benzylcyclopropylaminocarbonyl)-5-(2-chlorphenyl)-10-methyl-7H-cyclopenta[4,5]thieno-[3,2-f][1,2,4]-triazolo-[4,3-a][1,4]diazepin

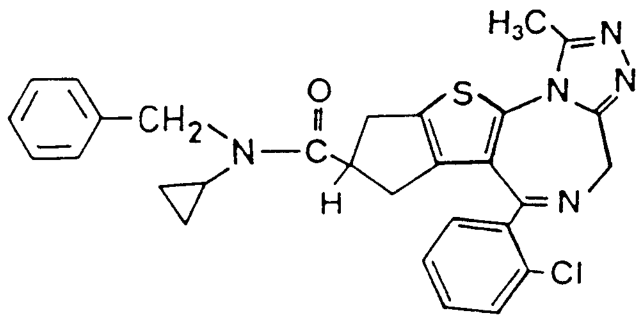

$[\alpha]_D$ = -27.8 (1 %, Methanol)
$^1$H-NMR ($CDCl_3$):δ = 7.46-7.02 (9H, m, aryl-H); 4.90 (2H, s, breit, $CH_2$-7-ring), 4.56 (2H, AB-system, N-$CH_2$-aryl); 4.26 (1H, m, O=C-CH); 3.24 (2H, m $CH_2$(2)); 2.70 (3H, s, $CH_3$-triazol); 2.51-2.03 (3H, m, $CH_2$(4)); 0.93-0.62 (4H, m, ($CH_2$-$CH_2$ cyclopropan).

Beispiel 10

s(-)-3-(Dibenzylaminocarbonyl)-5-(2-chlorphenyl)-10-methyl-7H-cyclopenta[4,5]thieno-[3,2-f][1,2,4]-triazolo-[4,3-a][1,4]diazepin

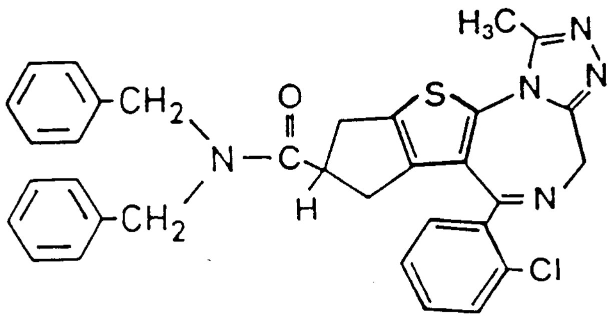

$[\alpha]_D$ = -27.8° (1 %, Methanol)
$^1$H-NMR ($CDCl_3$):δ = 7.45-6.96 (14H, m, aryl-H); 4.89 (2H, s, breit, $CH_2$-7-ring), 4.57; 4.39 (4H, 2-AB-system, N-($CH_2$(2)); 3.88-2.99 (3H, m, O=C-CH-$CH_2$(2)); 2.69 (3H, s, $CH_3$-triazol); 2.58-1.98 (2H, m, $CH_2$(4)-).

Beispiel 11

S(-)-3-(Benzylallylaminocarbonyl)-5-(2-chlorphenyl)-10-methyl-7H-cyclopenta[4,5]thieno-[3,2-f][1,2,4]-triazolo-[4,3-a][1,4]diazepin

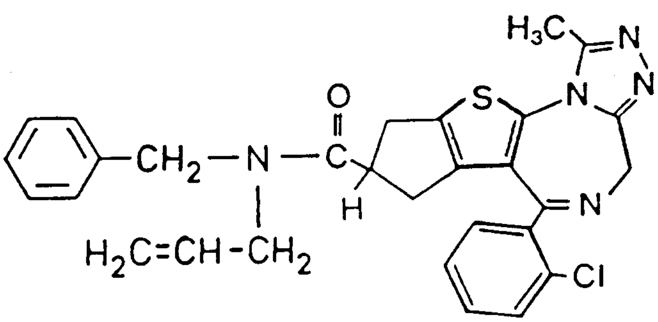

$[\alpha]_D$ = -21.1° (1 %, Methanol)
$^1$H-NMR (DMSO-d6):δ = 7.45-7.05 (9H, m, aryl-H); 5.65 (1H, m, H=C); 5.03 (2H, m, =$CH_2$); 4.80 (2H, AB-system, $CH_2$-7-ring); 4.46 (2H, AB-System, N-$CH_2$-aryl); 4,84 (3H, m, O=C-CH, N-$CH_2$-CH=); 3.15 (2H, m, $CH_2$(2)); 2.59 (3H, s, $CH_3$-triazol); 2.18 (2H, m, $CH_2$(4)).

Beispiel 12

(+/-)-3-(Benzylpropargylaminocarbonyl)-5-(2-chlorphenyl)-10-methyl-7H-cyclopenta[4,5]thieno-[3,2-f][1,2,4]-triazolo-[4,3-a][1,4]diazepin

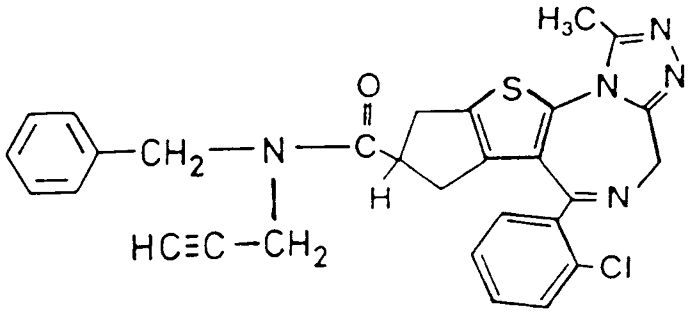

$^1H$-NMR ($CDCl_3$):$\delta$ = 7.46-7.00 (9H, m, aryl-H); 4.87 (2H, s, breit, $CH_2$-7-ring), 4.19 (2H, AB-system, N-$CH_2$-C≡); 4.62; 4.64 (2H, AB-system, N-$CH_2$-aryl); 3.75-2.98 (3H, m, O=C-CH-$CH_2$(2)); 2.70; 2.68 (3H, 2s, $CH_3$-triazol); 2.62-1.92 (2H, m, $CH_2$(4)); 2.26; 2,21 (1H, 2t, J=1Hz, HC≡C).

Beispiel 13

S(-)-3-((4-Fluorbenzyl)propylaminocarbonyl)-5-(2-chlorphenyl)-10-methyl-7H-cyclopenta[4,5]thieno-[3,2-f]-[1,2,4]-triazolo-[4,3-a][1,4]diazepin

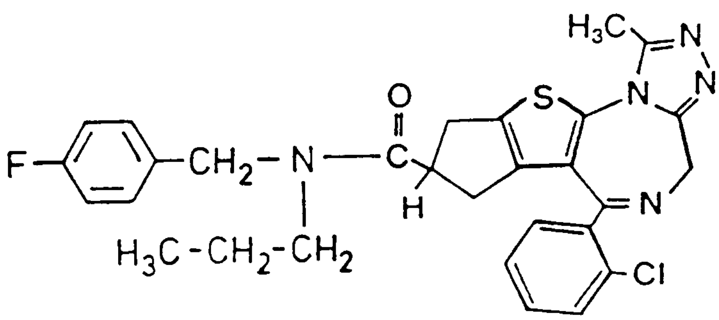

$[\alpha]_D$ = -21.3° (1 %, Methanol)
$^1H$-NMR ($CDCl_3$):$\delta$ = 7.45-6.88 (8H, m, aryl-H); 4.89 (2H, s, breit, $CH_2$-7-ring), 4.51, 4.45 (2H, 2-AB-system, N-$CH_2$-aryl); 3.86-2.92 (5H, m, N-$CH_2CH_2CH_3$, O=C-CH-$CH_2$(2)); 2.70; 2.68 (3H, 2s, $CH_3$-triazol); 2.51-2.03 (2H, m, $CH_2$(4)); 1.50 (2H, m, N-$CH_2CH_2CH_3$); 0.85 (3H, m, N-$CH_2CH_2$-$CH_3$).

Beispiel 14

S(-)-3-((4-Chlorbenzyl)propylaminocarbonyl)-5-(2-chlorphenyl)-10-methyl-7H-cyclopenta[4,5]thieno-[3,2-f]-[1,2,4]-triazolo-[4,3-a][1,4]diazepin

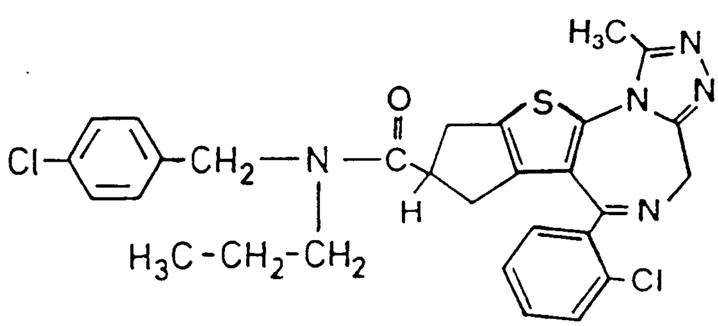

$[\alpha]_D$ = -21.1° (1 %, Methanol)
$^1H$-NMR (DMSO-d6):$\delta$ = 7.46-7.03 (8H, m, aryl-H); 4.80 (2H, AB-system, N-$CH_2$-7-ring); 4.46 (2H, AB-

System, N-$CH_2$-aryl); 3.83 (1H, m, O=C-CH); 3.17 (4H, m, N-$CH_2CH_2$-$CH_3$; $CH_2$(2)); 2.59 (3H, s, $CH_3$-triazol); 2.15 (2H, m, $CH_2$(4)); 1.43 (2H, m, N-$CH_2$-$CH_2CH_3$); 0.77 (3H, m, N-$CH_2CH_2CH_2$).

Beispiel 15

S(-)-3-((4-Methylbenzyl)aminocarbonyl)-5-(2-chlorphenyl)-10-methyl-7H-cyclopenta[4,5]thieno-[3,2-f][1,2,4]-triazolo-[4,3-a][1,4]diazepin

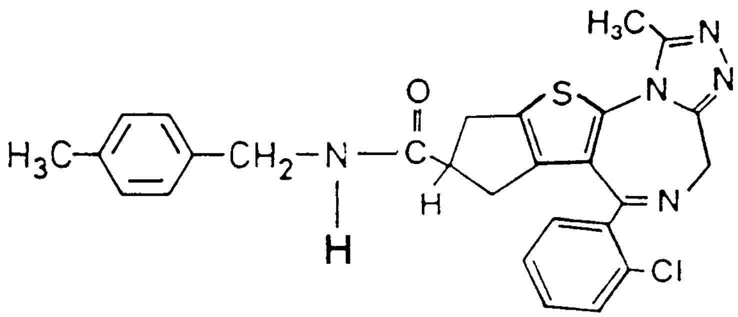

$[\alpha]_D$ = -35.6° (1 %, Methanol)
$^1$H-NMR ($CDCl_3$):$\delta$ = 7.45-7.03 (8H, m, aryl-H); 6.24 (1H, t, J=6Hz, NH); 4.82 (2H, s, breit, $CH_2$-7-ring), 4.34 (2H, C.$CH_2$-aryl); 3.44-3.07 (3H, m, O=C-CH-$CH_2$(2)); 2.64 (3H, s, $CH_3$-triazol); 2.33 (3H, s, $CH_3$-aryl); 2.50 - 2.02 (2H, m, $CH_2$(4)).

Beispiel 16

S(-)-3-((4-Methylbenzyl)methylaminocarbonyl)-5-(2-chlorphenyl)-10-methyl-7H-cyclopenta[4,5]thieno-[3,2-f]-[1,2,4]-triazolo-[4,3-a][1,4]diazepin

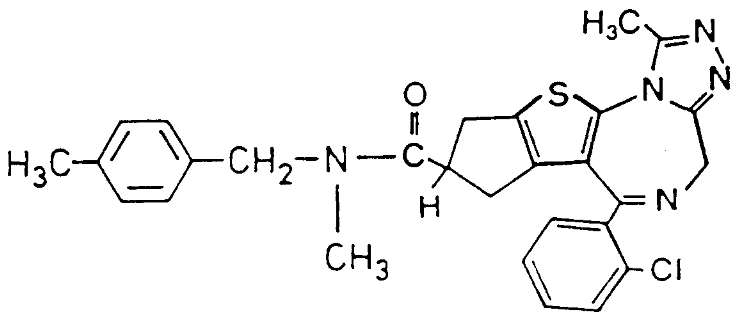

$[\alpha]_D$ = -31,4° (1 %, Methanol)
$^1$H-NMR ($CDCl_3$) :$\delta$ =7.45-6.89 (8H, m, aryl-H); 4.87 (2H, s, breit, $CH_2$-7-ring); 7.51, 4.44 (2H, 2AB-system, N-$CH_2$-aryl); 3.88 - 2.93 (3H, m, O=CH-$CH_2$(2)); 2.93, 2,87 (3H, 2s, N-$CH_3$); 2.70, 2.68 (3H, 2s, $CH_3$-triazol); 2.37, 2.34 (3H, 2s, $CH_3$-aryl); 2.55 - 2.02 (2H, m, $CH_2$(4)).

Beispiel 17

S(-)-3-((4-Trifluormethylbenzyl)methylaminocarbonyl)-5-(2-chlorphenyl)-10-methyl-7H-cyclopenta[4,5]thieno-[3,2-f][1,2,4]-triazolo-[4,3-a][1,4]diazepin

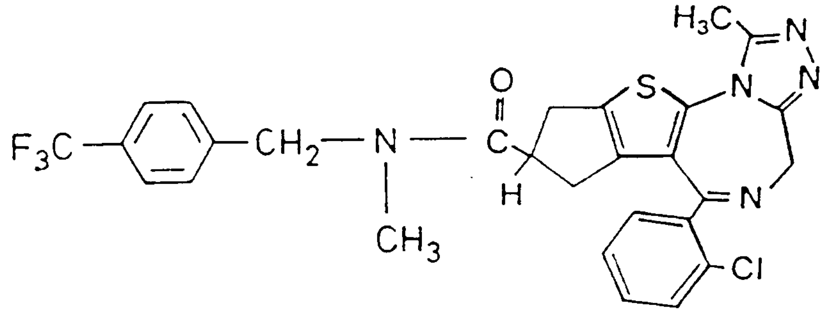

$[\alpha]_D$ = -33,0° (1 %, Methanol).
$^1$H-NMR ($CDCl_3$):$\delta$ = 7.66 - 7.14 (8H, m, aryl-H); 4.91 (2H, s, $CH_2$-7-ring); 4.59, 4.61 (2H, 2AB-system, N-$CH_2$-aryl); 3.89 - 2.92 (3H, m, OC-CH-$CH_2$(2));2.95, 2.89 (3H, 2s, N-$CH_3$); 2.70, 2.68 (3H, 2s, $CH_3$-triazol); 2.56 - 2.04 (2H, m, $CH_2$(4)).

Beispiel 18

S(-)-3-((4-Trifluormethylbenzyl)propylaminocarbonyl)-5-(2-chlorphenyl)-10-methyl-7H-cyclopenta[4,5]thieno-[3,2-f][1,2,4]-triazolo-[4,3-a][1,4]diazepin

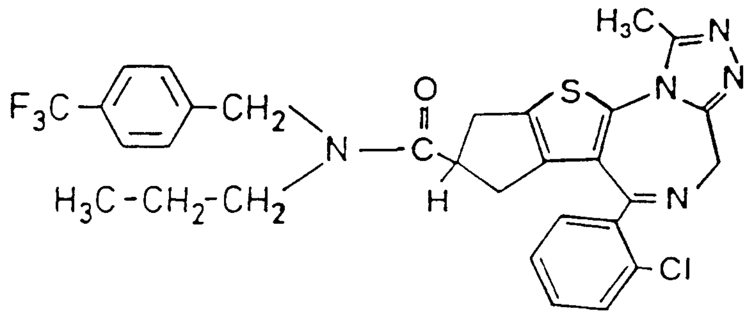

53 g (0.109 mol) des S(-)-3-Carboxy-5-(2-chlorphenyl)-10- methyl-7H-cyclopenta[4,5]thieno[3,2-f][1,2,4]-triazolo-[4,3-a][1,4]diazepins werden in 250 ml DMF vorgelegt.

Bei 20°C werden anschließend 18,3 g (0.12 mol) Hydroxybenzotriazol (HOBT), 23,7 g (0.109 mol) N-4-Trifluormethylbenzyl-N-n-propylamin und 25,6 g (0.125 mol) Dicyclohexylcarbodiimid (DCCI) und weitere 250 ml Dimethylformamid (DMF) zugegeben. Man läßt 24 Stunden nachreagieren. Danach wird auf 5°C gekühlt und filtriert. Das Filtrat wird mit 500 ml Wasser versetzt und mit Essigester extrahiert. Die gesammelten organischen Phasen werden mit Wasser und gesättigter Natriumhydrogencarbonatlösung gewaschen, mit Magnesiumsulfat getrocknet und über Kieselgur/Kohle filtriert. Nach üblicher Aufarbeitung erhält man durch Kristallisation aus Ethanol/Diisopropylether (1 : 1) 50 g farblose Kristalle der Titelverbindung. Die $^3$H-PAF-Rezeptorbindung - bestimmt unter Verwendung humaner Thrombozyten - beträgt $K_i$ = 1,8 nMol/ltr. (Die Methode ist beispielsweise in der Europäischen Patentanmeldung 368 175 beschrieben).
$[\alpha]_D$ = -26,3° (1 %, Methanol); Fp. 144°C.
$^1$H-NMR ($CDCl_3$):$\delta$ = 7.64 - 7.14 (8H, m, aryl-H); 4.87 (2H, s, breit, $CH_2$-7-ring); 4.61, 4.55 (2H, 2AB-system, n-$CH_2$-aryl); 3.90 - 2.95 (5H, m, OC-CH-$CH_2$, N-$CH_2$-$CH_2$-$CH_3$); 2.69, 2.67 (3H, 2s, $CH_3$-triazol); 2.50 - 1.93 (2H, m, $CH_2$(4)); 1.52 (2H, m, N-$CH_2$-$CH_2$-$CH_3$); 0.85 (3H, m, N-$CH_2$-$CH_2$-$CH_3$).

Die Synthese der als Ausgangsverbindung genannten Carbonsäure ist aus dem Stand der Technik bekannt, beispielsweise aus der Europäischen Patentanmeldung 388 789.

Beispiel 18a

(+/-)-3-((4-Trifluormethylbenzyl)propylaminocarbonyl)-5-(2-chlorphenyl-10-methyl-7H-cyclopenta[4,5]thieno-[3,2-f][1,2,4]-triazolo-[4,3-a][1,4]diazepin

In Analogie Zu Beispiel 18 erhält man aus dem racemischen 3-Carboxy-5-(2-chlorphenyl)-10-methyl-7H-cyclopenta[4,5]thieno[3,2-f][1,2,4]triazolo-[4,3-a][1,4]diazepin und N-4-Trifluormethylbenzyl-N-n-propylamin die Titelverbindung als amorphes weißes Pulver.

Beispiel 18b

R(+)-3-((4-Trifluormethylbenzyl)propylaminocarbonyl)-5-(2-chlorphenyl)-10-methyl-7H-cyclopenta[4,5]thieno-[3,2-f][1,2,4]-triazolo-[4,3-a][1,4]diazepin

Die Titelverbindung ist in Analogie zu Beispiel 18 aus der entsprechenden R(+)-Carbonsäure erhältlich. $[\alpha]_D$ = + 26,0° (1 %, Methanol).

Beispiel 19

S(-)-3-((4-Methylsulfonylbenzyl)propylaminocarbonyl)-5-(2-chlorphenyl)-10-methyl-7H-cyclopenta[4,5]thieno-[3,2-f][1,2,4]-triazolo-[4,3-a][1,4]diazepin

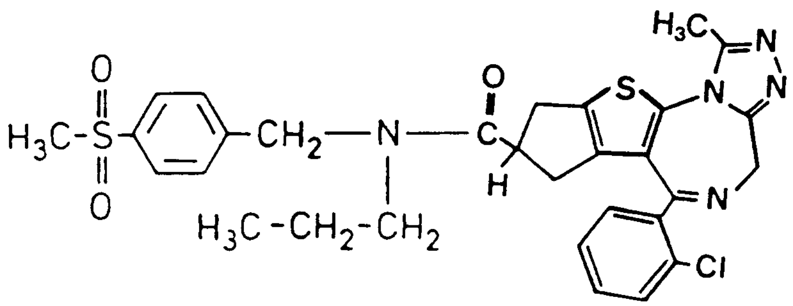

$[\alpha]_D$ = -27,8° (1 %, Methanol).
$^1$H-NMR ($CDCl_3$):$\delta$ = 7.95 - 7.21 (8H, m, aryl-H); 4.92 (2H, s, breit, $CH_2$-7-ring); 4.63, 4.57 (2H, 2AB-system, N-$CH_2$-aryl); 3.92 - 2.98 (5H, m, O=C-CH-$CH_2$(2), N-$CH_2$-$CH_2$-$CH_3$); 3.07, 3.05 (3H, 2s, $SO_2CH_3$); 2.70, 2.68 (3H, 2s, $CH_3$-triazol); 2.54 - 2.10 (2H, m, $CH_2$(4)); 1.52 (2H, m, N-$CH_2$-$CH_2$-$CH_3$); 0.85 (3H, m, N-$CH_2$-$CH_2$-$CH_3$).

Beispiel 20

(+/-)-3-((2-Hydroxybenzyl)propylaminocarbonyl)-5-(2-chlorphenyl)-10-methyl-7H-cyclopenta[4,5]thieno-[3,2-f][1,2,4]-triazolo-[4,3-a][1,4]diazepin

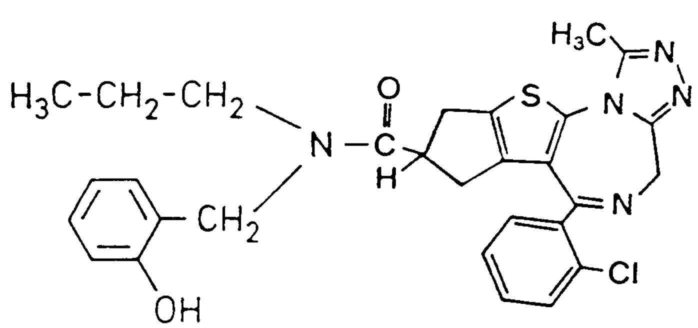

$^1$H-NMR ($CDCl_3$):$\delta$ = 5.47 - 6.74 (8H, m, aryl-H); 4.88 (2H, s, breit, $CH_2$-7-ring); 4.42 (2H, AB-System, N-$CH_2$-aryl); 3.73 - 3.01 (5H, m, O=C-$CH_2$(2), N-$CH_2$-$CH_2$-$CH_3$); 2.46 (3H, s, $CH_3$-triazol); 2.50 - 1.96 (2H, m, $CH_2$(4), 1.58 (2H, m, N-$CH_2$-$CH_2$-$CH_3$); 0.90 (3H, m, N-$CH_2$-$CH_2$-$CH_3$); 9.40 (1H, s, OH).

Beispiel 21

(+/-)-3-(Phenethylpropylaminocarbonyl)-5-(2-chlorphenyl)-10-methyl-7H-cyclopenta[4,5]thieno-[3,2-f][1,2,4]-triazolo-[4,3-a][1,4]diazepin

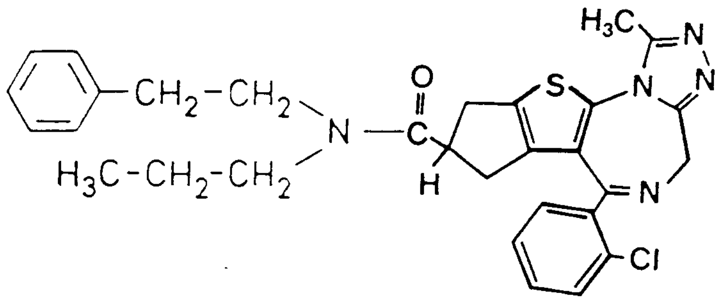

$^1$H-NMR ($CDCl_3$):$\delta$ = 7.47 - 6.99 (9H, m, aryl-H); 4.84 (2H, s, breit, $CH_2$-7-ring); 3.67 - 2.73 (9H, m, N-$CH_2$-$CH_2$-aryl, N-$CH_2$-$CH_2$-$CH_3$, O=C-CH-$CH_2$(2)); 2.69, 2.68 (3H, 2s, $CH_3$-triazol); 2.46 - 1.83 (2H, m, $CH_2$(4)); 1.50 (2H, m, N-$CH_2$-$CH_2$-$CH_3$); 0.85 (3H, m, N-$CH_2$-$CH_2$-$CH_3$).

Beispiel 22

S(-)-3-((2-Furfurylmethyl)propylaminocarbonyl)-5-(2-chlorphenyl)-10-methyl-7H-cyclopenta[4,5]thieno-[3,2-f]-[1,2,4]-triazolo-[4,3-a][1,4]diazepin

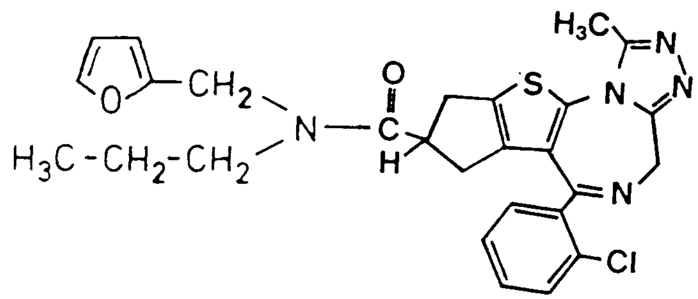

$[\alpha]_D$ = -14.5° (1 %, Methanol)
$^1$H-NMR ($CDCl_3$):$\delta$ = 7.46 - 6.11 (7H, m, aryl-H, furan-H); 4.88 (2H, s, breit, $CH_2$-7-ring); 4.52, 4.37 (2H, 2AB-system, N-$CH_2$-furan); 3.90 - 3.05 (5H, m, O=C-CH-$CH_2$(2), N-$CH_2$-$CH_2$-$CH_3$); 2.69 (3H, s, $CH_3$-triazol); 2.55 - 1.96 (2H, m, $CH_2$(4)); 1.46 (2H, m, N-$CH_2$-$CH_2$-$CH_3$); 0.83 (3H, m, N-$CH_2$-$CH_2$-$CH_3$).

Beispiel 23

S(-)-3-((2-Thienylmethyl)propylaminocarbonyl)-5-(2-chlorphenyl)-10-methyl-7H-cyclopenta[4,5]thieno-[3,2-f]-[1,2,4]-triazolo-[4,3-a][1,4]diazepin

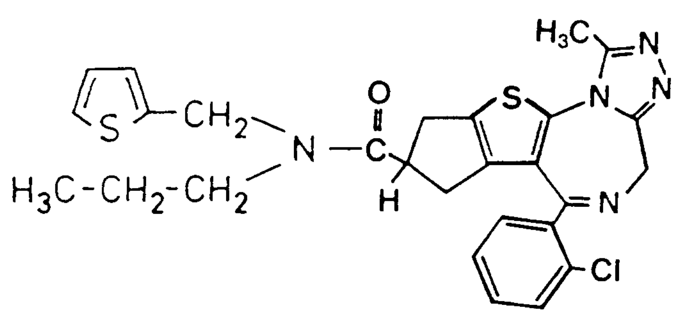

$[\alpha]_D$ = -25.8° (1 %, Methanol)

$^1$H-NMR ($CDCl_3$):δ = 7.43 - 6.78 (7H, m, aryl-H, thiophen-H); 4.89 (2H, s, breit, $CH_2$-7-ring); 4.67, 4.59 (2H, 2AB-system, N-$CH_2$-thiophen); 3.74 - 3.14 (5H, m, O=C-CH-$CH_2$(2), N-$CH_2$-$CH_2$-$CH_3$); 2.70 (3H, s, $CH_3$-triazol); 2.57 - 1.95 (2H, m, $CH_2$(4));1.52 (2H, m, N-$CH_2$-$CH_2$-$CH_3$); 0.86 (3H, m, N-$CH_2$-$CH_3$).

Beispiel 24

R(+)-3-((4-Trifluormethylbenzyl)methylaminocarbonyl)-5-(2-chlorphenyl)-10-methyl-7H-cyclopenta[4,5]-thieno-[3,2-f][1,2,4]-triazolo-[4,3-a][1,4]diazepin

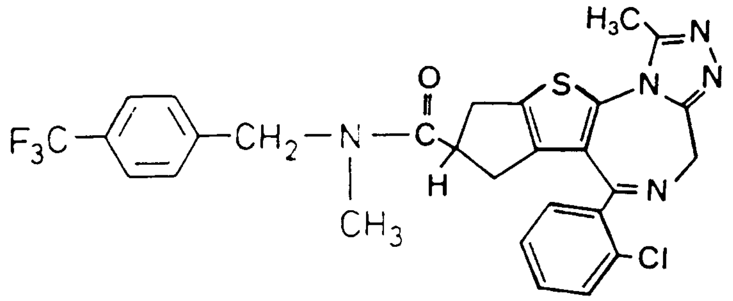

$[\alpha]_D$ = +30.2° (1 %, Methanol)
$^1$H-NMR ($CDCl_3$):δ = 7.65 - 7.09 (8H, m, aryl-H); 4.88 (2H, s, breit, $CH_2$-7-ring); 4.61, 4.56 (2H, 2AB-system, N-$CH_2$-aryl); 3.90 - 2.98 (3H, m, O=C-CH-$CH_2$(2)); 2.95, 2.90 (3H, 25, N-$CH_3$); 2.70, 2.68 (3H, 2s, $CH_3$-triazol); 2.54 - 2.07 (2H, m, $CH_2$(4)).

Beispiel 25

(+/-)-3-(Benzylmethylaminocarbonyl)-5-(2-chlorphenyl)-10-methyl-7H-cyclopenta[4,5]thieno-[3,2-f][1,2,4]-triazolo-[4,3-a][1,4]diazepin

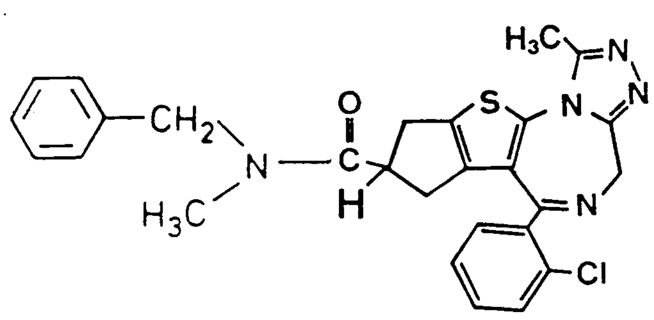

$^1$H-NMR ($CDCl_3$):δ = 7.47 - 7.00 (9H, m, aryl-H); 4.92 (2H, s, breit, $CH_2$-7-ring); 4.56, 4.50 (2H, 2AB-system, N-$CH_2$-aryl); 3.88 - 2.93 (3H, m, O=C-CH-$CH_2$(2)); 2.94, 2.86 (3H, 25, N-$CH_3$); 2.71, 2.69 (3H, 2s, $CH_3$-triazol); 2.55 - 2.02 (2H, m, $CH_2$(4)).

Beispiel 26

(+/-)-3-(Benzylpropylaminocarbonyl)-5-(2-chlorphenyl)-10-methyl-7H-cyclopenta[4,5]thieno-[3,2-f][1,2,4]-triazolo-[4,3-a][1,4]diazepin

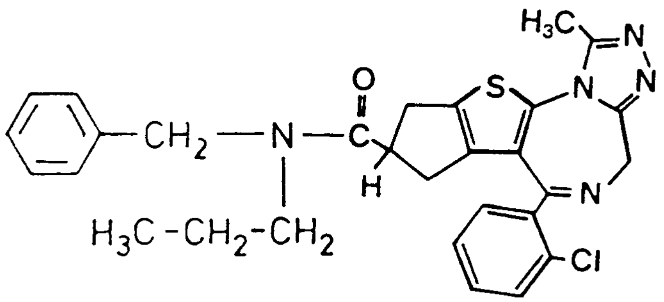

$^1$H-NMR ($CDCl_3$):$\delta$ = 7.47 - 7.00 (9H, m, aryl-H); 4.90 (2H, s, breit, $CH_2$-7-ring); 4.56, 4.47 (2H, 2AB-system, N-$CH_2$-aryl); 3.88 - 2.93 (5H, m, O=C-CH-$CH_2$(2), N-$CH_2$-$CH_2$-$CH_3$); 2.70, 2,68 (3H, 2s, $CH_3$-triazol); 2.57 - 1.80 (2H, m, $CH_2$(4)); 1.52 (2H, m, N-$CH_2$-$CH_2$-$CH_3$); 0.85 (3H, m, N-$CH_2$-$CH_2$-$CH_3$).

Beispiel 27

(+/-)-3-(Benzylmethylaminocarbonyl)-5-(2-chlorphenyl)-7H-cyclopenta[4,5]thieno-[3,2-f][1,3]imidazolo-[1,2-a][1,4]diazepin

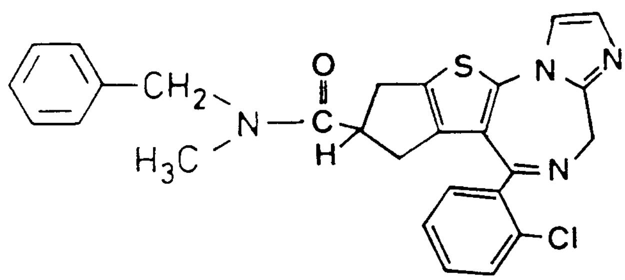

$^1$H-NMR (DMSO-d6):$\delta$ = 7.45 - 7.00 (11H, m, aryl-H, imidazol-H); 4.70 (2H, AB-System, $CH_2$-7-ring); 4.46 (2H, s, N-$CH_2$-aryl); 2.81 (3H, s, N-$CH_3$); 3.83 (1H, m, O=C-CH); 3.12 (2H, m, $CH_2$(2)); 2.15 (2H, m, $CH_2$(4)-).

Beispiel 28

(+/-)-4-(Benzylmethylaminocarbonyl)-11-methyl-6-(2-Chlorphenyl)-2,3,4,5-tetrahydro-8H-[1]-benzothieno-[3,2-f][1,3]imidazo-[1,2-a][1,4]-diazepin

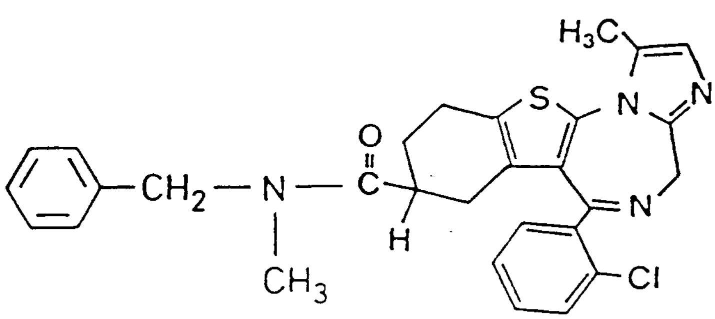

$^1$H-NMR (DMSO-d6):$\delta$ = 7.45 - 7.02 (9H, m, aryl-H); 6.78 (1H, qu, J=0,5Hz, HC); 4.42 (2H, AB-system, N-$CH_2$-aryl); 4.68 (2H, s, breit, $CH_2$-7-ring); 2.93 - 2.69 (3H, m, O=C-CH, $CH_2$(2)); 2.38 (3H, d, J=0,5Hz, $CH_3$-imidazol); 2.07 - 1.67 (4H, m, $CH_2$(3), $CH_2$(5).
Schmp.: 159-160 °C

Beispiel 29

(+/-)-3-(4-((4-Trifluormethylphenyl)-ethano)-benzylpropylamino-carbonyl)-5-(2-chlorphenyl)-10-methyl-7H-cyclopenta-[4,5]thieno-[3,2-f][1,2,4]-triazolo-[4,3-a][1,4]diazepin

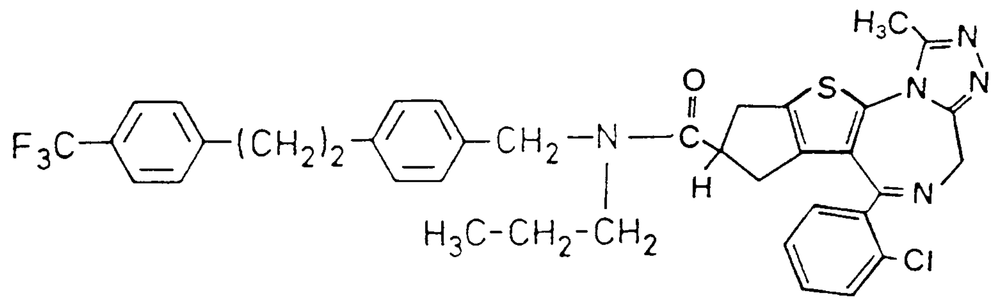

$^1$H-NMR (DMSO-d6):δ = 7.62 - 6.95 (12H, m, aryl-H); 4.47 (2H, AB-system, N-$CH_2$-aryl); 4.41 (1H, m, CH-7-ring); 3.83 (1H, m, O=C-CH); 3.28 - 2.40 (10H, m, aryl-$CH_2$-$CH_2$), N-$CH_2$-$CH_2$-$CH_3$, $CH_2$(2), $CH_2$(4)); 1.40 (2H, m, N-$CH_2$-$CH_2$-$CH_3$); 1.89 (3H, d, J=6Hz, CH-$CH_3$); 0.74 (3H, m, N-$CH_2$-$CH_2$-$CH_3$).

Beispiel 30

(+/-)-3-(4-(2-(3,4,5-Trimethoxyphenyl)ethano)benzylamino-carbonyl)-5-(2-chlorphenyl)-10-methyl-7H-cyclopenta-[4,5]thieno-[3,2-f][1,2,4]-triazolo-[4,3-a][1,4]diazepin

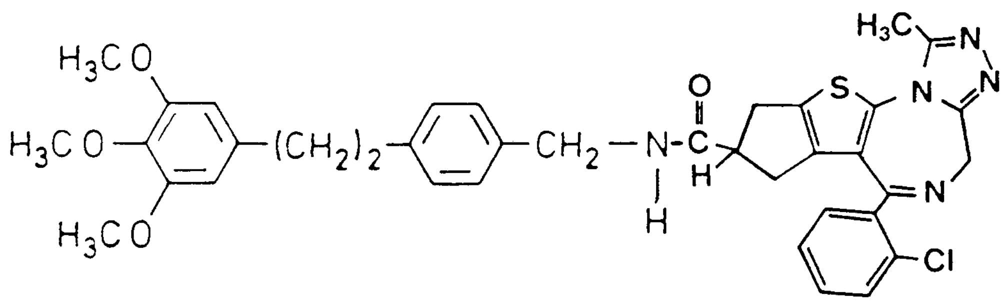

$^1$H-NMR (DMSO-d6):δ = 7.57 - 6.97 (12H, m, aryl-H); 4.98 (2H, AB-system, $CH_2$-7-ring); 4.43 (2H, AB-system, N-$CH_2$-aryl); 3.83 (1H, m, O=C-CH); 3.26 - 2.55 (8H, m, aryl-$CH_2$-$CH_2$, N-$CH_2$-$CH_2$-$CH_3$, $CH_2$(2)); 2.59 (3H, s, $CH_3$-triazol); 2.20 (2H, m, $CH_2$(4)); 1.40 (2H, m, N-$CH_2$-$CH_2$-$CH_3$); 0.75 (3H, m, N-$CH_2$-$CH_2$-$CH_3$).

Beispiel 31

3-((4-Hydroxyphenethyl)aminocarbonyl)-5-(2-chlorphenyl)-10-methyl-7H-cyclopenta[4,5]thieno-[3,2-f][1,2,4]-triazolo-[4,3-a][1,4]diazepin

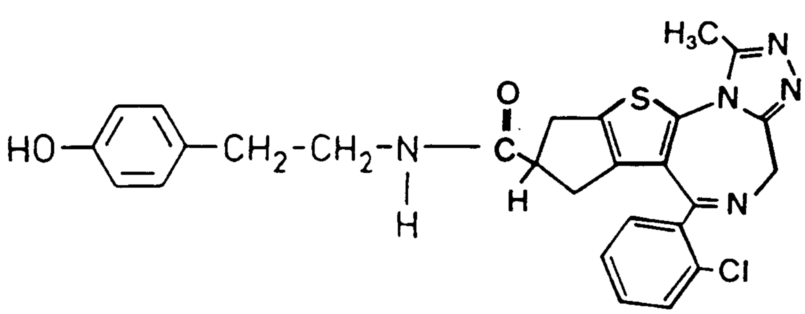

$^1$H-NMR (DMSO-d6):δ = 7.93 (1H, t, J=7Hz, NH); 7.52 - 6.59 (8H, m, aryl-H); 9.26 (1H, s, OH); 3.61 - 2.93

(7H, m, O = C-CH-$CH_2$(2), N-$CH_2$-$CH_2$); 4.76 (2H, s, breit, $CH_2$-7-ring); 2.61 (3H, s, $CH_3$-triazol); 2.09 (2H, m, $CH_2$(4)).

Beispiel 32

4-(2-Chlorphenyl)-9-methyl-thieno-[3,2-f][1,2,4]-triazolo-[4,3-a][1,4]diazepino-2-(2-ethano)-carbonsäure-(4-fluorbenzyl)propylamid

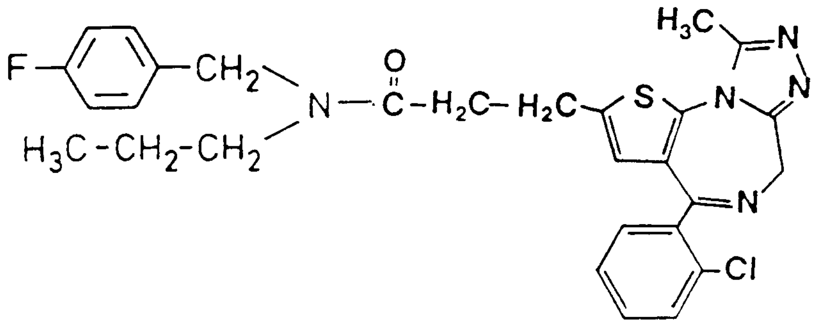

$^1$H-NMR ($CDCl_3$):δ = 7.45 - 6.89 (8H, m, aryl-H); 6.43, 6.37 (1H, 2s, thiophen-H); 4.92 (2H, s, $CH_2$-7-ring); 4.53, 4.45 (2H, 2s, N-$CH_2$-aryl); 3.39 - 2.55 (6H, m, O = C-$CH_2$-$CH_2$, N-$CH_2$-$CH_2$-$CH_3$); 2.69 (3H, s, $CH_3$-triazol); 1.52 (2H, m, N-$CH_2$-$CH_2$-$CH_3$); 0.86 (3H, m, N-$CH_2$-$CH_2$-$CH_3$).
Schmp.: 176-178 ° C

Beispiel 33

4-(2-Chlorphenyl)-9-methyl-thieno-[3,2-f][1,2,4]-triazolo-[4,3-a][1,4]diazepino-2-(2-ethano)carbonsäure-(4-trifluormethylbenzyl)propylamid

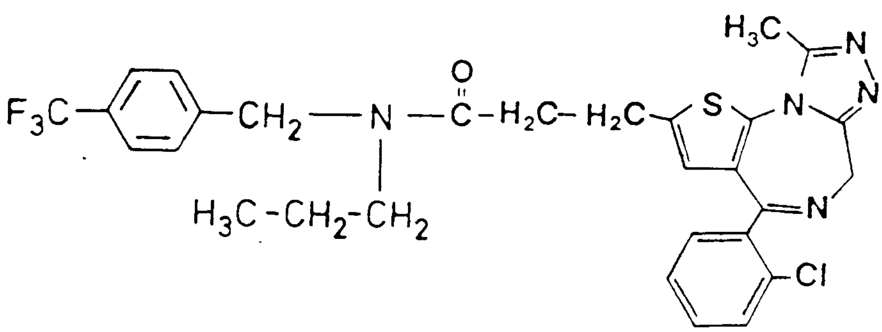

$^1$H-NMR ($CDCl_3$):δ = 7.62 - 7.19 (8H, m, aryl-H); 6.45, 6.38 (1H, 2s, thiophen-H); 4.92 (2H, s, $CH_2$-7-ring); 4.63, 4.56 (2H, 2s, N-$CH_2$-aryl); 3.40 - 2.52 (6H, m, O = C-$CH_2$-$CH_2$, N-$CH_2$-$CH_2$-$CH_3$); 2.69 (3H, s, $CH_3$-triazol); 1.54 (2H, m, N-$CH_2$-$CH_2$-$CH_3$); 0.88 (3H, m, N-$CH_2$-$CH_2$-$CH_3$).
Schmp.: 182-183 ° C

Beispiel 34

4-(2-Chlorphenyl)-9-methyl-thieno-[3,2-f][1,2,4]-triazolo-[4,3-a][1,4]diazepino-2-(2-ethano)carbonsäure-(4-trifluormethylbenzyl)methylamid

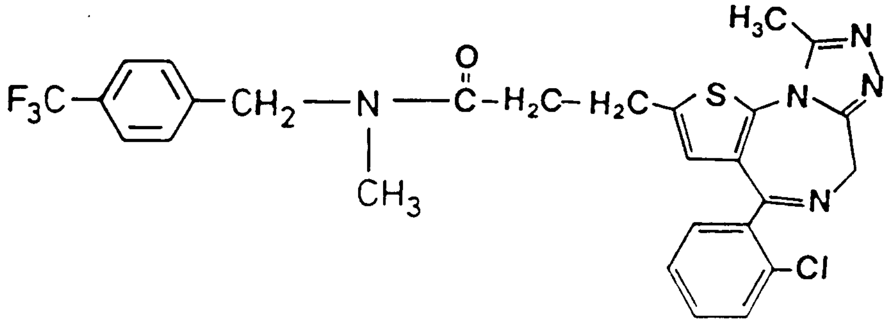

$^1$H-NMR ($CDCl_3$):$\delta$ = 7.64 - 7.14 (8H, m, aryl-H); 6.46, 6.39 (1H, 2s, thiophen-H); 4.93 (2H, s, $CH_2$-7-ring); 4.64, 4.56 (2H, 2s, N-$CH_2$-aryl); 3.18 (2H, m, N-$CH_2$); 2.99, 2.93 (3H, 22, N-$CH_3$); 2.73, 2.70 (3H, 2s, $CH_3$-triazol); 2.72 (2H, m, $CH_2$-thiophen).

Beispiel 35

4-(2-Chlorphenyl)-9-methyl-thieno-[3,2-f][1,2,4]-triazolo-[4,3-a][1,4]diazepino-2-(2-ethano)carbonsäure-(4-methylsulfonylbenzyl)propylamid

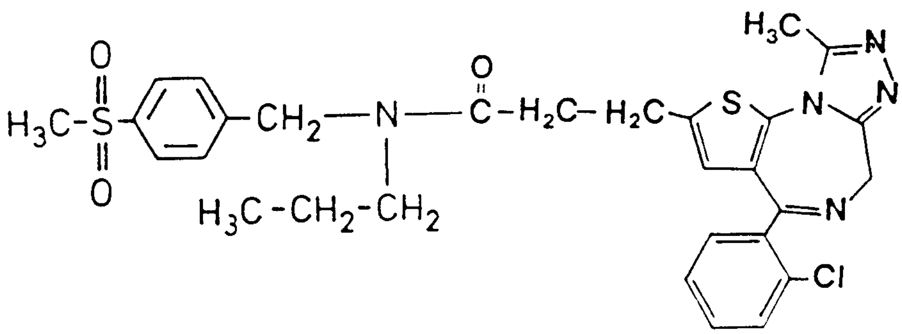

$^1$H-NMR ($CDCl_3$):$\delta$ = 7.93 - 7.21 (8H, m, aryl-H); 6.45, 6.38 (1H, 2s, thiophen-H); 4.93 (2H, s, $CH_2$-7-ring); 4.67, 4.59 (2H, 2s, N-$CH_2$-aryl); 3.40 - 2.50 (6H, m, O=C-$CH_2$-$CH_2$, N-$CH_2$-$CH_2$-$CH_3$); 3.05, 5.02 (3H, 2s, $SO_2$-$CH_3$); 2.70 (3H, s, $CH_3$-triazol); 1.54 (2H, m, N-$CH_2$-$CH_2$-$CH_3$); 0.87 (3H, m, N-$CH_2$-$CH_2$-$CH_3$).
Schmp.: 172-174 ° C

Beispiel 36

4-(2-Chlorphenyl)-9-methyl-thieno-[3,2-f][1,2,4]-triazolo-[4,3-a][1,4]diazepino-2-(2-ethano)carbonsäure-(4-(4-trifluormethylphenyl)ethano)benzylpropylamid

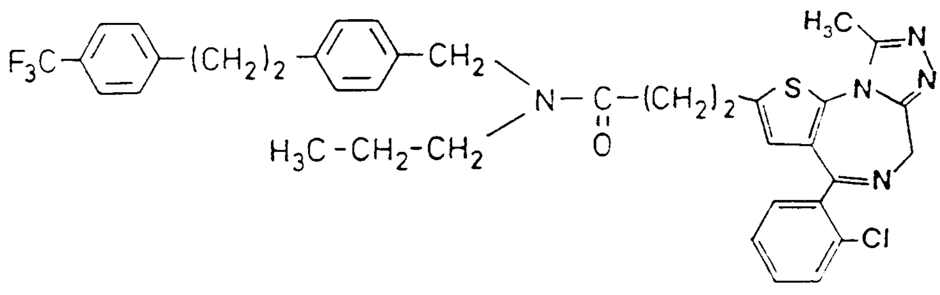

$^1$H-NMR (DMSO-d6):$\delta$ = 7.57 - 7.08 (12H, m, aryl-H); 6.45 (1H, s, thiophen-H); 4.78 (2H, s, $CH_2$-7-ring); 4.45 (2H, s, N-$CH_2$-aryl); 3.17 - 2.66 (10H, m, O=C-$CH_2$-$CH_2$, N-$CH_2$-$CH_2$-$CH_3$, aryl-$CH_2$-$CH_2$); 2.57 (3H, s,

$CH_3$-triazol); 1.42 (2H, m, N-$CH_2$-$CH_2$-$CH_3$); 0.76 (3H, m, N-$CH_2$-$CH_2$-$CH_3$).

Beispiel 37

( + /-)-3-Benzylpropylaminocarbonyl-5-(2-chlorphenyl)-7,10-dimethyl-7H-cyclopenta[4,5]thieno-[3,2,-f][1,2,4]-triazolo-[4,3-a][1,4]diazepin

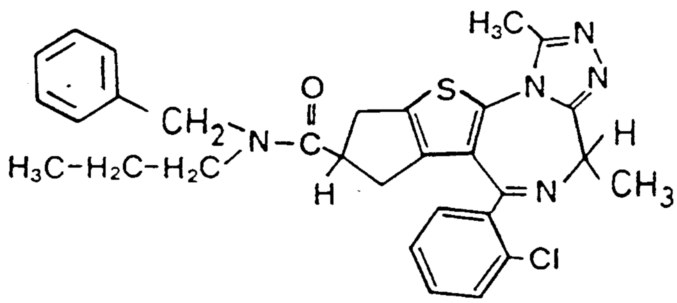

$^1$H-NMR ($CDCl_3$): $\delta$ = 7.48 - 6.98 (9H, m, aryl-H); 4.54, 4.52 (2H, 2-AB-systems, N-$CH_2$-aryl); 4.38, 4.32 1H, 2 qu, J = 7Hz, N-CH-C=N); 3.86 - 2.98 (5H, m, N-$CH_2CH_2CH_3$; CH-C=0; thiophen-$CH_2$-2); 2.69, 2.67 (3H, 25, $CH_3$-triazol); 2.55 - 1.90 (2H, m, thiophen-$CH_2$-3); 2.03, 1.85 (3H, 2d, J = 7Hz, $CH_3$-CH); 1.52 (2H, m, N-$CH_2CH_2CH_3$); 0.95 (3H, m, N-$CH_2CH_2CH_3$).

Beispiel 38

( + /-)-3-((4-Trifluormethylbenzyl)propylaminocarbonyl)-5-(2-chlorphenyl)-7,10-dimethyl-7H-cyclopenta[4,5]-thieno-[3,2-f][1,2,4]triazolo[4,3-a][1,4]diazepin

Ausgehend von 3-Carboxy-5-(2-chlorphenyl)-7,10-dimethyl-7H-cyclopenta-[4,5]thieno[3,2-f][1,2,4]-triazolo-[4,3-a][1,4]-diazepin - herstellbar aus der entsprechenden 3-Methoxycarbonyl-Verbindung = Europäische Patentanmeldung 368 175 - insbesondere Beispiel 9 - durch Verseifung - erhält man durch Umsetzung mit N-4-Trifluormethylbenzyl-N-n-propylamin in Analogie zu Beispiel 18 die Titelverbindung.

Beispiel 39

3-S(-)- ((4-Trifluormethylbenzyl)propylaminocarbonyl)-5-(2-chlorphenyl)-7,10-dimethyl-7H-cyclopenta[4,5]-thieno[3,2-f][1,2,4]triazolo[4,3-a][1,4]diazepin

Ausgehend von S(-)-3-Carboxy-5-chlorphenyl-7,10-dimethyl-7H-cyclopenta[4,5]thieno[3,2-f][1,2,4]-triazolo[4,3-a][1,4]diazepin in Analogie zu Beispiel 38 erhält man die Titelverbindung.

Die beiden Enantiomeren können wie in der Europäischen Patentanmeldung 368 175 unter Verwendung optisch aktiver Säulen getrennt werden.

Man erhält das 3-S(-)-((4-Trifluormethylbenzyl)propylaminocarbonyl-5-(2-chlorphenyl9-7S-7,10-dimethyl-7H-cyclopenta[4,5]thieno[3,2-f][1,2,4]triazolo[4,3-a] [1,4]diazepin und das

3-S(-)-((4-Trifluormethylbenzyl)propylaminocarbonyl-5-(2-chlorphenyl)-7R-7,10-dimethyl-7H-cyclopenta-[4,5]thieno[3,2-f][1,2,4]triazolo[4,3-a][1,4]diazepin.

Beispiel 40

( + /-)-3-((4-Trifluormethylbenzyl)propyylaminocarbonyl-5-(2-chlorphenyl)-7,7,10-trimethyl-cyclopenta[4,5]-thieno[3,2-f][1,2,4]triazolo[4,3-a][1,4]diazepin

Ausgehend von 3-Carboxy-5-(2-chlorphenyl-7,7,10-trimethyl-cyclopenta[4,5]thieno[3,2-f][1,2,4]triazolo-[4,3-a][1,4]diazepin erhält man die Titelverbindung in Analogie zu Beispiel 18.

Die neuen Verbindungen der allgemeinen Formel Ia können topisch, oral, parenteral transdermal oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Sirupe, Suppositorien usw. Eine wirksame Dosis der erfindungsgemäßen Verbindungen liegt bei oraler Anwendung zwischen 1 und 50, vorzugsweise zwischen 3 und 20 mg/Dosis, bei intravenöser oder intramuskulärer Anwendung zwischen 0,01 und 50, vorzugsweise zwischen 0,1 und 10 mg/Dosis. Für die Inhalation sollen Lösungen, die 0.01 bis 1,0, vorzugsweise 0.1 bis 0.5 % Wirkstoff enthalten, eingesetzt werden.

Die folgenden Beispiele dienen zur näheren Erläuterung der Erfindung

Beispiel 1

Tabletten, enthaltend 10 mg Substanz der Formel I

Zusammensetzung:

| | |
|---|---|
| Substanz der Formel I | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45°C getrocknet. Das trockne Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

Beispiel 2

Dragées, enthaltend 5 mg Substanz der Formel I

Zusammensetzung:

| | |
|---|---|
| Substanz B der Formel I | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C

und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.
Dragéegewicht: 130 mg

Beispiel 3

Tabletten, enthaltend 50 mg Substanz der Formel I

Zusammensetzung:

| | |
|---|---|
| Substanz B der Formel I | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:
Die Substanz Calciumphosphat, Milchzucker und Maisstärke werden mit einer wässerigen Polyvinylpyrrolidonlösung gleichmäßig befeuchtet. Die Masse wird durch ein Sieb mit 2 mm Maschenweite gegeben, im Umlufttrockenschrank bei 50°C getrocknet und erneut gesiebt. Nach Zumischen des Schmiermittels wird das Granulat auf einer Tablettiermaschine gepreßt.

Beispiel 4

Kapseln, enthaltend 50 mg Substanz der Formel I

Zusammensetzung:

| | |
|---|---|
| Substanz der Formel I | 50,0 mg |
| Maisstärke | 268,5 mg |
| Magnesiumstearat | 1,5 mg |
| | 320,0 mg |

Herstellung:
Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

Beispiel 5

Suppositorien, enthaltend 50 mg Substanz der Formel I

Zusammensetzung:

| | |
|---|---|
| Substanz der Formel I | 50 mg |
| Adeps solidus | 1.650 mg |
| | 1.700 mg |

Herstellung:

Das Hartfett wird geschmolzen. Bei 40 ° C wird die gemahlene Wirksubstanz homogen dispergiert. Es wird auf 38 ° C abgekühlt und in schwach vorgekühlte Suppositorienformen ausgegossen.

Beispiel 6

Orale Suspension, enthaltend 50 mg Substanz der Formel I pro 5 ml

Zusammensetzung:

| | |
|---|---|
| Substanz der Formel I | 50 mg |
| Hydroxyethylcellulose | 50 mg |
| Sorbinsäue | 5 mg |
| Sorbit 70%ig | 600 mg |
| Glycerin | 200 mg |
| Aroma | 15 mg |
| Wasser ad | 5 ml |

Herstellung:

Destilliertes Wasser wird auf 70 ° C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.

**Patentansprüche**

**1.** Neue Thienodiazepine der allgemeinen Formel I

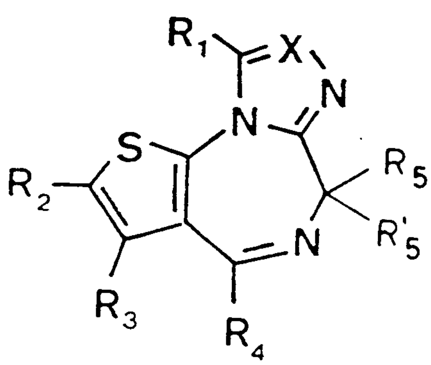

worin

$R_1$ Wasserstoff, Halogen, eine verzweigte oder unverzweigte Alkylgruppe mit 1 bis 4 Kohlenstoffatom(en), bevorzugt Methyl, die gegebenenfalls durch Hydroxy oder Halogen substituiert sein kann, eine Cyclopropylgruppe, eine Cyclobutylgruppe, eine Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;

$R_2$ den Rest -Z-$R_a$

Z eine verzweigte oder unverzweigte Alkyl-, Alkenyl- oder Alkinylgruppe mit n Kohlenstoffatomen, wobei Z gegebenenfalls zusätzlich durch einen Arylrest oder zusätzlich durch $R_a$ substituiert sein kann;

n eine der Zahlen 0, 1, 2, 3, 4, 5, 6, 7, 8, 9 oder 10, wobei im Falle Z einen Alkenyl- oder Alkinylrest bedeutet, n >1 ist und bevorzugt die Bedeutung 2, 3 und 4 besitzt;

$R_3$ Wasserstoff oder Methyl

oder

$R_2$ und $R_3$ zusammen einen Rest der allgemeinen Formel

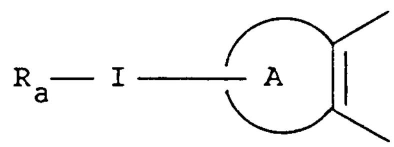

worin

A einen ankondensierten einfach ungesättigten 5-, 6- oder 7-gliedrigen Ring,

I eine verzweigte oder unverzweigte Alkyl-, Alkenyl- oder Alkinylgruppe mit m Kohlenstoffatomen;

m 0, 1 2, 3, 4, 5 oder 6, bevorzugt O;

$R_a$ einen Rest der allgemeinen Formel

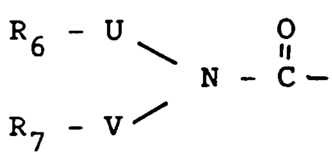

worin
U und V unabhängig voneinander, gleich oder verschieden eine Einfachbindung, eine verzweigte oder unverzweigte $C_1$-$C_6$, bevorzugt $C_1$-$C_3$-Alkylgruppe, eine $C_2$-$C_6$, bevorzugt $C_2$-$C_3$-Alkenylgruppe, oder eine $C_2$ bis $C_6$, bevorzugt $C_3$-$C_4$-Alkinylgruppe bedeuten können,

$R_6$ Wasserstoff oder einen Rest der Formel

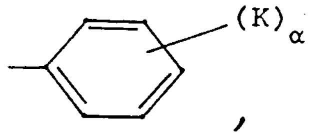

,

gegebenenfalls substituiertes $C_3$-$C_6$-Cycloalkyl- oder $C_5$-$C_6$-Cycloalkenyl;

$R_7$ einen Rest der Formel

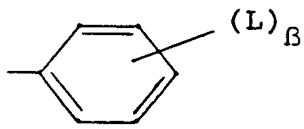

oder

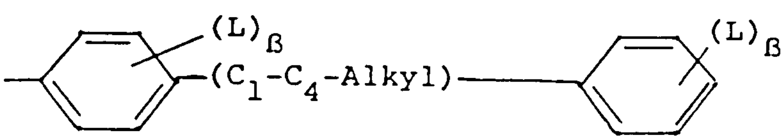

,

gegebenenfalls substituiertes $C_3$-$C_6$-Cycloalkyl oder $C_5$-$C_6$-Cycloalkenyl;

K und L unabhängig voneinander Wasserstoff, Halogen, Hydroxy, Nitro, Cyano, Trifluormethyl, gegebenenfalls durch Halogen oder Hydroxy substituiertes, verzweigtes oder unverzweigtes Alkyl mit 1 bis 8 - bevorzugt 1 bis 4 - Kohlenstoffatom(en), gegebenenfalls substituiertes $C_3$-$C_6$-Cycloalkyl, gegebenenfalls substituiertes, unverzweigtes oder verzweigtes Alkoxy mit 1 bis 8 bevorzugt 1 bis 4 Kohlenstoffatom(en), einen Rest der Formel

$-(F)_{0-1}-E-(F)_{0-1}-H$, $(F)_{0-1}-NO_2$,

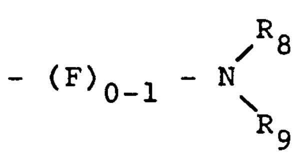

,

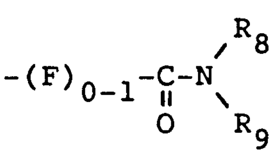

,

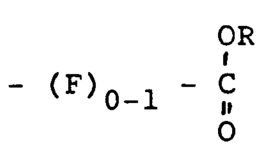

R = $C_1$ - $C_4$-Alkyl,

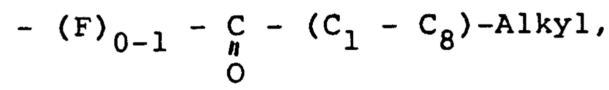

bevorzugt $C_1$ - $C_4$-Alkyl,

worin F eine verzweigte oder unverzweigte Alkylgruppe mit 1 bis 4 Kohlenstoffatom(en), bevorzugt 1 bis 2 Kohlenstoffatom(en)

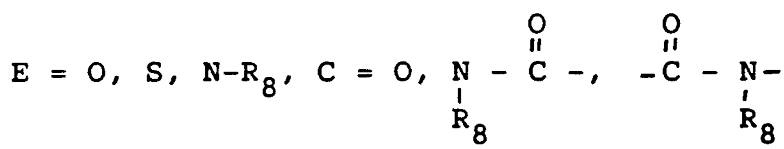

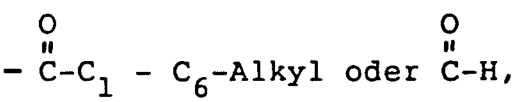

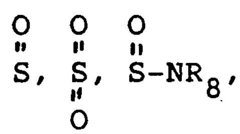

F eine verzweigte oder unverzweigte gegebenenfalls substituierte Alkylgruppe mit 1 bis 8 - bevorzugt 1 bis 4 - Kohlenstoffatom(en), eine gegebenenfalls substituierte Alkenylgruppe mit 2 bis 8 - bevorzugt 2 bis 4 - Kohlenstoffatomen, eine gegebenenfalls substituierte Alkinylgruppe mit 2 bis 8 - bevorzugt 2 bis 4 Kohlenstoffatomen;

worin $R_8$ und $R_9$, die gleich oder verschieden sein können, Wasserstoff, Phenyl, substituiertes Phenyl, eine verzweigte oder unverzweigte Alkyl-, Alkenyl- oder Alkinylgruppe mit 1 bis 6 Kohlenstoffatomen, bevorzugt mit 1 bis 4 Kohlenstoffatom(en), die gegebenenfalls durch Halogen, Hydroxy, Nitro, Amino, substituiertes Amino, $C_3$-$C_6$-Cycloalkyl, bevorzugt Cyclopropyl, $C_1$-$C_4$-Alkoxy, bevorzugt Methoxy;

$R_8$ oder $R_9$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring, einen gegebenenfalls substituierten $C_3$- bis $C_7$-Cycloalkylrest, einen gegebenenfalls substituierten Cycloalkenylrest; oder

$R_8$ und $R_9$, sofern strukturell möglich, zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatom(en), bevorzugt Methyl, substituiert sein kann;

$\alpha$ und $\beta$ unabhängig voneinander eine der Zahlen 1, 2, 3, 4 oder 5, wobei bei $\alpha$ oder $\beta$ größer 1 alle K und L gleich, teilweise gleich oder verschieden sein können;

$R_6$ ein C-verknüpfter gesättigter oder ungesättigter 5- oder 6-gliedriger Heterocyclus, der als Heteroatome Stickstoff, Sauerstoff oder Schwefel enthalten kann, bevorzugt ein Pyridyl-, Thiophen- oder Furanrest, der gegebenenfalls ein- oder mehrfach, gleich oder verschieden durch Halogen, $C_1$-$C_4$-Alkyl,

$C_1$-$C_4$-Halogenalkyl, bevorzugt $CF_3$, Nitro, $NR_8R_9$, S-$C_1$-$C_4$-Alkylsubstituiert sein kann,

$R_7$ ein C-verknüpfter gesättigter oder ungesättigter 5- oder 6-gliedriger Heterocyclus, der als Heteroatome Stickstoff, Sauerstoff oder Schwefel enthalten kann, bevorzugt ein Pyridyl-, Thiophen- oder Furanrest, der gegebenenfalls ein- oder mehrfach, gleich oder verschieden durch Halogen, $C_1$-$C_4$-Alkyl, $C_1$-$C_4$-Halogenalkyl, bevorzugt $CF_3$, Nitro, $NR_8R_9$, S-$C_1$-$C_4$-Alkyl substituiert sein kann,

$R_4$ Phenyl, wobei der Phenylring ein- oder mehrfach, bevorzugt in 2-Stellung, durch Methyl, bevorzugt Halogen, besonders bevorzugt Chlor oder Brom, Nitro, Alkoxy, bevorzugt Methoxy und/oder Trifluormethyl substituiert sein kann, Pyridyl oder Thienyl, das gegebenenfalls durch $C_1$-$C_4$-Alkyl oder Halogen substituiert sein kann;

$R_5$ Wasserstoff, Methyl, Trifluormethyl, Cyclopropyl, Hydroxymethyl;
$R'_5$ Methyl, bevorzugt Wasserstoff,
X Stickstoff, C-H, C-$CH_3$;

bedeuten kann,

gegebenenfalls in Form ihrer Racemate, ihrer Enantiomere, ihrer Diastereomere und ihrer Gemische sowie gegebenenfalls ihrer physiologisch unbedenklichen Säureadditionssalze.

**2.** Thienodiazepine der allgemeinen Formel Ia

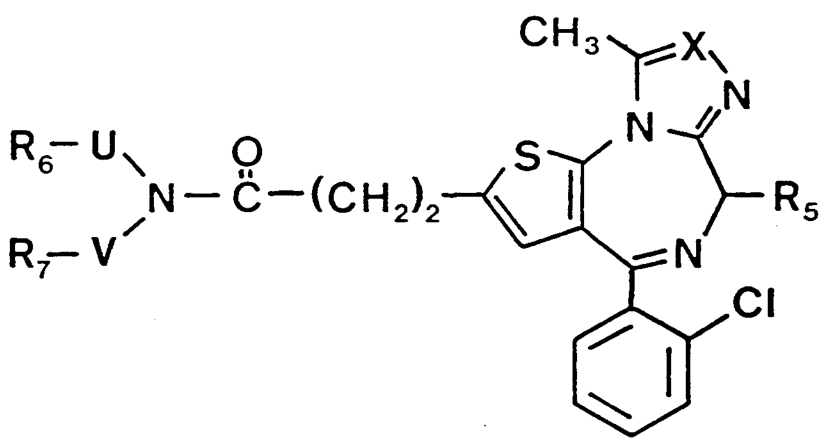

Ia

worin

U eine Einfachbindung, verzweigtes oder unverzweigtes $C_1$-$C_4$-Alkyl, $C_3$ oder $C_4$ Alkenyl, $C_3$ oder $C_4$-Alkinyl;

V eine Einfachbindung, verzweigtes oder unverzweigtes $C_1$-$C_4$-Alkyl

$R_5$ Wasserstoff oder Methyl;

$R_6$ einen Rest der Formel

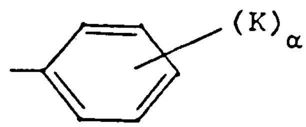

wie zuvor definiert, Cyclopropyl, Cyclopentyl, Cyclohexyl, bevorzugt Wasserstoff;

$R_7$ einen Rest der Formel

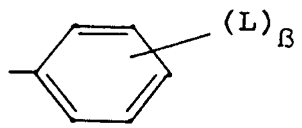

,

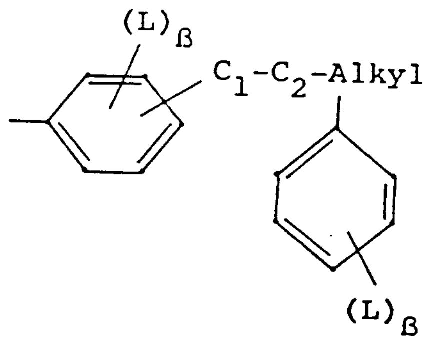

wie zuvor definiert, ein Pyridinyl-, ein Thien-2-yl, ein Furan-2-ylrest und

X Stickstoff oder CH bedeuten können.

**3.** Thienodiazepine in Form ihrer Racemate sowie ihrer in Form ihrer optisch aktiven Isomeren der allgemeinen Formel Ib und Ic:

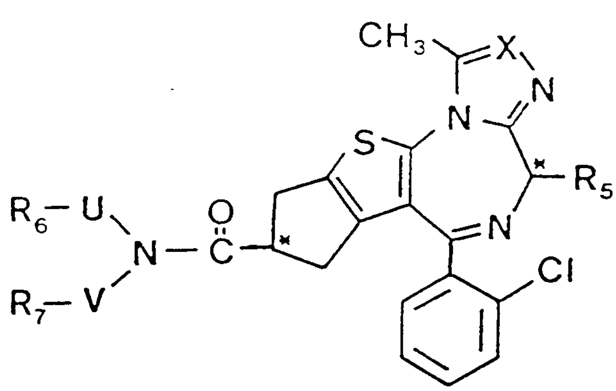

Ib

und

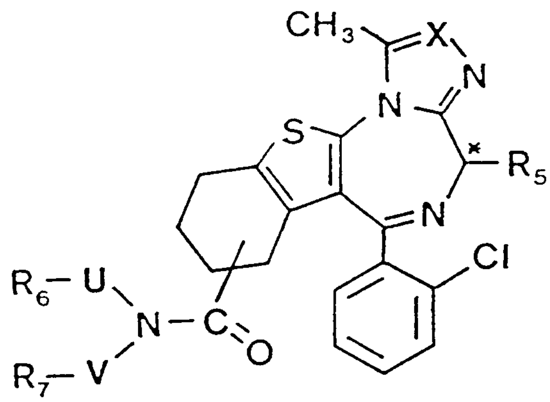

Ic

worin

U eine Einfachbindung, verzweigtes oder unverzweigtes $C_1$-$C_4$-Alkyl, $C_3$ oder $C_4$ Alkenyl, $C_3$ oder $C_4$-Alkinyl;

V eine Einfachbindung, verzweigtes oder unverzweigtes $C_1$-$C_4$-Alkyl

$R_5$ Wasserstoff oder Methyl;

$R_6$ einen Rest der Formel

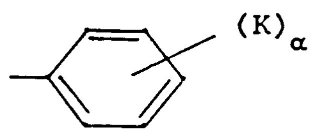

wie vor definiert, Cyclopropyl, Cyclopentyl, Cyclohexyl, bevorzugt Wasserstoff;

$R_7$ einen Rest der Formel

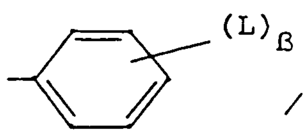

/

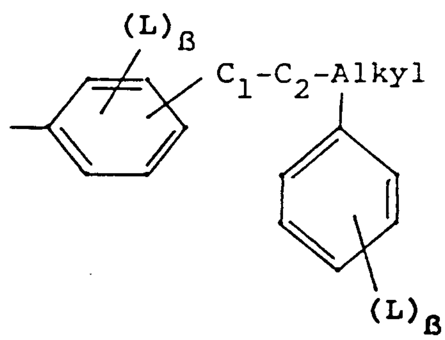

wie zuvor definiert, ein Pyridinyl-, ein Thien-2-yl- oder ein Furan-2-ylrest und

X Stickstoff oder CH bedeuten können.

**4.** Thienodiazepine der allgemeinen Formel Ia, Ib und Ic, worin X Stickstoff bedeutet,

U eine Einfachbindung oder $C_1$-$C_4$-Alkyl, Propyl, Allyl, Propargyl;

V $C_1$-$C_4$-Alkyl, $CH_2$;

$R_6$ Wasserstoff, Cyclopropyl oder

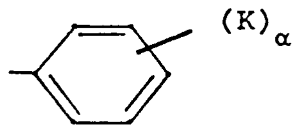

R 7

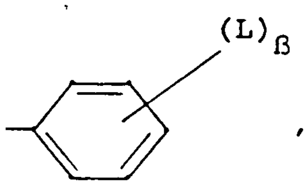

,

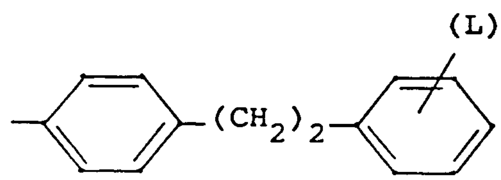

K und L unabhängig voneinander Wasserstoff, gegebenenfalls durch Halogen, Hydroxy, substituiertes $C_1$-$C_4$-Alkyl, $C_2$-$C_4$-Alkinyl, oder $C_2$-$C_4$-Alkenyl, Nitro, Cyano, Hydroxy, $C_1$-$C_4$-Alkoxy, $C_1$-$C_4$-Alkoxy-$C_1$-$C_4$-alkyl, $C_1$-$C_4$-Alkylsulfonyl, Halogen, $C_1$-$C_4$-Hydroxyalkyl-sulfonyl, $COOC_1$-$C_4$-Alkyl, $COOC_{1-4}$-Alkylphenyl, Cyclopropyl, SH, S-$C_1$-$C_4$-Alkyl;

$\alpha$ 1, 2 oder 3, bevorzugt 1;
$\beta$ 1, 2 oder 3, bevorzugt 1;
bevorzugt
$(K)_\alpha$ Wasserstoff, $CF_3$ oder Halogen und
$(L)_\beta$ bevorzugt Wasserstoff, F, Cl, Br, Methyl, Trifluormethyl, $CH_3O_2S$, Hydroxy, wobei $(K)_\alpha$ und $(L)_\beta$ bevorzugt in der 4-Position des Phenylringes angeordnet ist.

**5.** Thienodiazepine in Form ihrer S(-)-Isomeren der allgemeinen Formel Ib, worin $R_5$ Wasserstoff bedeutet und alle anderen Substituenten die zuvor angegebene Bedeutung besitzen können.

**6.** Pharmazeutische Zubereitung, gekennzeichnet durch einen Gehalt an einer Verbindung nach einem der Ansprüche 1 bis 5.

**7.** Verwendung von Verbindungen nach einem der Ansprüche 1 bis 5 bei der Herstellung von pharmazeutischen Zubereitungen zur Behandlung von Erkrankungen, die durch endogen gebildetes PAF induziert werden.

**8.** Verwendung von Verbindungen nach einem der Ansprüche 1 bis 5 als Arzneimittel.

**9.** Verfahen zur Herstellung von Thienodiazepinen der allgemeinen Formel I wie in Anspruch 1 oder 2 definiert dadurch gekennzeichnet, daß man eine Verbindung der allgemeinen Formel I, worin Ra = COOH bedeutet,

a) mit einem Amin der allgemeinen Formel

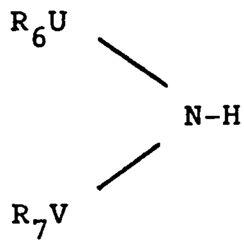

worin $R_6$, $R_7$, U und V die in Anspruch 1, 2, 3 oder 4 genannte Bedeutung aufweisen

umsetzt, oder daß man

b) die Carbonsäure der allgemeinen Formel II in üblicher Weise in ein säurehalogenid oder ein Säureanhydrid überführt und dieses anschließend mit dem gewünschten Amin umsetzt.